# EUROPEAN PATENT SPECIFICATION

(11) **EP 2 187 959 B1**
(45) Date of publication and mention of the grant of the patent: **10.04.2013**
(21) Application number: 08788692.5
(22) Date of filing: 18.08.2008
(51) Int. Cl.: A61K 39/04, G01N 33/569

(54) **DIAGNOSTIC METHOD AND KIT**
DIAGNOSEVERFAHREN UND KIT
PROCEDE ET COFFRET DE DIAGNOSTIC

(30) Priority: 17.08.2007 GB 0716070; 15.08.2008 IE 20080670
(43) Date of publication of application: 26.05.2010
(73) Proprietor: Fusion Antibodies Limited, Belfast BT17 0QL (GB); Enfer Technology Limited, Co. Tipperary (IE)
(72) Inventor: OLWILL, Shane A., Belfast BT17 0QL (GB); JOHNSTON, James A., Belfast BT17 0QL (GB); KWOK, Hang Fai, Belfast BT17 0QL (GB); WHELAN, Clare, Co. Tipperary (IE); CLARKE, John, Co. Tipperary (IE)
(74) Representative: Main, Malcolm Charles
(86) International application number: PCT/GB2008/050721
(87) International publication number: WO 2009/024822

(56) References cited:
- WO-A-2006/117538
- VORDERMEIER ET AL: "Progress in the development of vaccines and diagnostic reagents to control tuberculosis in cattle" VETERINARY JOURNAL, BAILLIERE TINDALL, LONDON, GB, vol. 171, no. 2, 1 March 2006 (2006-03-01), pages 229-244, XP005293200 ISSN: 1090-0233
- PINXTEREN VAN L A H ET AL: "Diagnosis of Tuberculosis Based on the Two Specific Antigens ESAT-6 and CFP10" CLINICAL AND DIAGNOSTIC LABORATORY IMMUNOLOGY, AMERICAN SOCIETY FOR MICROBIOLOGY, US, vol. 7, no. 2, 1 March 2000 (2000-03-01), pages 155-160, XP002252151 ISSN: 1071-412X
- LIU ET AL: "A novel fusion protein-based indirect enzyme-linked immunosorbent assay for the detection of bovine tuberculosis" TUBERCULOSIS, ELSEVIER, GB, vol. 87, no. 3, 11 April 2007 (2007-04-11), pages 212-217, XP022020620 ISSN: 1472-9792
- LYASHCHENKO K P ET AL: "PrimaTB STAT-PAK assay, a novel, rapid lateral-flow test for tuberculosis in nonhuman primates" CLINICAL AND VACCINE IMMUNOLOGY 200709 US, vol. 14, no. 9, September 2007 (2007-09), pages 1158-1164, XP002511617 ISSN: 1556-6811
- WATERS W R ET AL: "Antibody responses in reindeer (Rangifer tarandus) infected with Mycobacterium bovis" CLINICAL AND DIAGNOSTIC LABORATORY IMMUNOLOGY 200506 US, vol. 12, no. 6, June 2005 (2005-06), pages 727-735, XP002511618 ISSN: 1071-412X
- LYASHCHENKO K P ET AL: "A multi-antigen print immunoassay for the development of serological diagnosis of infectious diseases" JOURNAL OF IMMUNOLOGICAL METHODS, ELSEVIER SCIENCE PUBLISHERS B.V.,AMSTERDAM, NL, vol. 242, no. 1-2, 28 August 2000 (2000-08-28), pages 91-100, XP004210713 ISSN: 0022-1759
- LYASHCHENKO K P ET AL: "Novel monoclonal antibodies against major antigens of Mycobacterium bovis" SCANDINAVIAN JOURNAL OF IMMUNOLOGY 2001 GB, vol. 53, no. 5, 2001, pages 498-502, XP002511619 ISSN: 0300-9475

## Description

### Field of the Invention

The invention relates to methods and kits for the diagnosis and/or herd profiling of Mycobacterium infected or contaminated animals.

### Background to the Invention

Tuberculosis (TB), one of the most widespread infectious diseases, is the leading cause of death due to a single infectious agent among adults in the world. Mycobacterium tuberculosis is the most common cause of human TB.

However, an unknown proportion of cases of zoonotic tuberculosis are due to *M. bovis* with some due to *Mycobacterium avium.* Thus, infection of the animal population not only places a strain on economic resources but also presents a threat to human health. The requirement for successful diagnostic assays and potential vaccines has increased due to the recent rise in TB levels in the cattle population of countries such as Great Britain and the wild life reservoirs of the world.

Great Britain performs some 4.6 million tests on bovine TB, costing the tax payer £88m per annum (TB Conference *M. bovis* IV, Dublin 2005). Bovine TB varies regionally within GB, with the worst incidence seen in South Wales, Cornwall and Gloucestershire where 25% of all animals are infected. The incidence of TB is increasing at a rate of 2.5% per year in previously uninfected herds (TB Conference *M. bovis* IV, Dublin 2005).

In order to monitor and control the disease herd profiling is necessary. However, the methods currently used to monitor tuberculosis in animals suffer from a number of drawbacks. Nowadays, the disease control programmes for bovine TB carried out in most countries (e.g. US, Australia and GB etc.) are based on a test and removal strategy utilizing the intradermal skin test, which relies on PPD, a purified protein derivative of *M. bovis* strain AN5, to elicit an immune response in infected cattle.

In cattle, the intradermal skin tests currently used are the Caudal-fold Tuberculin Test (CFT) and the Comparative cervical tuberculin test (CCT). The Caudal-fold Tuberculin Test (CFT) is the primary screening test used to identify cattle herds potentially infected with bovine tuberculosis. It measures the immune response to Purified Protein Derivative (PPD) tuberculin *(M. bovis* AN5). If the animal's immune system recognizes the PPD, inflammatory cells (white blood cells) migrate to the injected site to help get rid of the foreign material (PPD). This cell mediated immune response may be recognized by swelling or discoloration at the site where PPD was injected. However, in 5% of cases, the CFT test may result in false-positive test results (due to exposure to or infection with other closely related bacteria, such as *M. avium and M. paratuberculosis)* or, in 15% of cases, in false-negative test results - where a very early stage of infection with bovine TB is not detected. As a follow up test to the CFT, the Comparative cervical tuberculin test (CCT) may be performed. The CCT test, in which suspect animals are injected with PPD avian and PPD bovine in the cervical (neck) region, is a more definitive test designed to determine if a response noted on the CFT test is more likely due to infection with *M. avium* or *M. bovis.* CCT Test-suspect cattle are subjected to further testing using necropsy and further diagnostic testing.

Disease control based on the skin test can be complemented by the gamma-interferon test, which measures the animals T cell response when exposed to PPD material. The gamma-interferon test is utilised as a second line diagnostic for 'skin test positive' animals (reports suggest it is more sensitive, but sometimes less specific than a skin test). It detects the cell-mediated immune response that develops following *M. bovis* infection (2 weeks).

The Gamma-Interferon test is not used as a primary test for mass screening on its own because it does not detect all skin test-positive infected animals; it is relatively expensive and is less specific than the CCT.

Due to their modes of action, the specificity of both the intradermal skin test and the gamma interferon test will always be an issue. The cross reactions induced by different mycobacteria strains and environmental mycobacteria such as *M. microti* and *M. africanum* and the conflicting requirements between specificity and sensitivity of the test antigens, all accrue to the difficulties in establishing a satisfactory serological protocol for bovine TB.

One of the problems associated with the complex antigenic nature of mycobacteria is the definition of those proteins which are important targets of the immune system and are thus likely present in large numbers of field samples. It is also important to recognize that there is regional variation in the infectious *M. bovis* strains.

An antibody assay developed using strain specific proteins could resolve both specificity and sensitivity issues.

A number of methods of discriminating between strains of tuberculosis have been suggested. US 6,686,166B, WO 2004/083448A, US2004/0063923A, and US 6,291,190B describe 129 genetic marker sequences which are suggested for use in the identification of strains of mycobacteria. However, as described herein the mere identification of such markers does not equate with practical utility in a diagnostic method. Many markers are not expressed sufficiently to reliably be used in the identification of a strain. Further, as described further below, the amount of expression of individual markers varies considerably, not only between strains, but within strains geographically and within strains dependent on the stage of infection.

US2005/0272104A suggests the use of the PPD antigens ESAT-6 and CFP-10 in the detection of Mycobacterium tuberculosis in humans. In general however, antibody (Ab) tests based upon PPD tuberculins are characterised by a low discriminating power, with the distribution of the antibody titers between infected and non-infected animals being widely overlapping (Amadori *et al.* 1998).

US 7,192,721 describes a test kit for detection of TB in a different non-primate mammals, the kit comprising a mycobacterial antigen cocktail comprising ESAT-6/CFP10 and 16kDa/MPB83 fusion proteins. In a sample of 85 cattle, the sensitivity demonstrated was 79%; however, in 513 cattle, the sensitivity was 73%.

Due to the heterogeneity of the disease, no single antigen is present in all cases of infection. However, although a great many antigens associated with tuberculosis infection have been identified in the prior art, to date, the use of particular combinations has not resulted in a test with an acceptable level of sensitivity and specificity to avoid the many false positive and false negative test results associated with conventional TB testing.

However, although relatively high sensitivity has been reported for some prior art tuberculosis kits, the sensitivity may only be maintained across selected populations of animals, for example within particular herds or in defined geographical regions, where variation in prevalent strains of tuberculosis is limited and thus variation between animals in their response to infection with such strains is limited. Presently available diagnostic kits do not provide specific combinations of antigens with sufficient coverage over different populations, herds and geographical areas for the reliable diagnosis of tuberculosis.

Although many antigens have been identified, by simply increasing the number of antigens in a test kit, the sensitivity and specificity of the test may not necessarily be improved. It is recognised in the art that, where diagnostic kits comprise too many antigenic additional antigens, the specificity and sensitivity of the kit may in fact be reduced. For example, US 792,721 describes a decrease in sensitivity and specificity associated with increasing the number of antigens used in the kits described therein. Thus, crucial to the design of an effective kit is the selection of antigens to be used therein.

As well as the problems discussed above with respect to identification of antigens for reliable diagnosis of tuberculosis infection, the formats of many diagnostic kits currently used may contribute to the lack of reliability. Cumulative results from animals experimentally infected with Mbv have shown there may be a transient antibody response to some antigens that occur at different stages of infection, further complicating development of a diagnostic assay (Amadori,et al 2002. Vet. Microbiol. 85:379-389). The advances that have been made show that some of the difficulties in developing an optimal assay can be overcome by including multiple antigens in the assay (Aagaard, et al 2006. J. Clin. Microbiol. 44:4326-4335., Amadori,et al 2002. Vet. Microbiol. 85:379-389). Although some success has also been made in identifying antigens potentially useful in multiplexed diagnostic assays (Aagaard, et al 2006. J. Clin. Microbiol. 44:4326-4335., Koo et al 2005. J. Clin. Microbiol. 43:4498-4506, Lightbody, et al 2000. Vet. Microbiol. 75:177-188, McNair et al 2001. Scand. J. Immunol. 53:365-371., Pollock, J. M. and P. Andersen. 1997. Infect. Immun. 65:2587-2592, Pollock, J. M. and P. Andersen. 1997. J. Infect. Dis. 175:1251-1254, Pollock, et al 2000. Vet. Rec. 146:659-665, Wiker et al 1998. Infect. Immun. 66:1445-1452). The necessity of using multiple antigens in an assay, has, however, introduced another challenge. Evaluation of the standard type of ELISA has shown sensitivity and specificity are reduced when multiple antigens are combined for analysis in a single well thus limiting the way a conventional ELISA can be used (Lyashchenko,et al 2000. J. Immunol. Methods 242:91-100). Fluorescence polarization has been evaluated as an alternative platform (Lin et all996. Clin. Diag. Lab. Immunol. 3:438-443, Surujballiet al 2002. Vet. Microbiol. 87:149-157). It has been successfully used in testing for *Mbv* in cattle, elk, llamas, and bison with a single antigen. A further improvement over an ELISA-based system has been the use of lateral flow technology (Koo et al 2005. J. Clin. Microbiol. 43:4498-4506, Lyashchenko,et al 2000. J. Immunol. Methods 242:91-100). This methodology has been used successfully for multiplexing antigens for visual analysis of patterns of antibody activity at the single animal level ( Greenwald, et al 2003. Diagn. Microbiol. Infect. Dis. 46:197-203, Lyashchenko, et al 2006. Clin. Vaccine Immunol. 13:722-732, Waters, et al 2006. Clin. Vaccine Immunol. 13:648-654, Waters, et a12006. Clin. Vaccine Immunol. 13:611-619, Wernery et al 2007. Vet. Microbiol. 122:108-115.). Other areas that have not been fully explored are electrochemiluminescence ( Lu, Y. et al 2006. J. Immunol. Methods 314:74-79, Yan, et al. 2004. J. Immunol. Methods 288:47-54) and chemiluminescence ( Liew et al 2007. Biotechniques 42:327-333). Commercial imaging systems using these technologies have been developed for multi-plexing and analysis of antibody activity to multiple antigens (Liew et al 2007. Biotechniques 42:327-333, Lu, Y. et al 2006. J. Immunol. Methods 314:74-79, Yan, et al. 2004. J. Immunol. Methods 288:47-54). The platforms are designed for rapid processing and analysis of large numbers of samples. However, a number of problems persist with many of these methods.

A test which can accurately and reliably diagnose the presence of tuberculosis infection with satisfactory sensitivity and specificity across different populations of animals has eluded the field.

### Summary of the Invention

In the broadest sense the present application is defined by the appended claims.

As described herein, the present inventors have overcome a number of problems with the prior art diagnostic methods and have identified a number of polypeptides, which together are useful for the sensitive specific diagnosis and profiling of Mycobacterium.

Each of the specific polypeptides is capable of eliciting a strong immune response in the absence of adjuvant. The characterized proteins / fragments which are immunogenic (or early antigens) and specific to one or other mycobacterial strains can be used reliably for the diagnosis of tuberculosis infection. As described in the examples, such polypeptides elicit a strong immune response in serum from animals immunised with *Mycobacterium bovis.* Significantly, the inventors have found that, when these polypeptides are used together in an assay, the sensitivity of tuberculosis testing can be vastly improved over the sensitivity obtained using prior art methods. Thus, the invention, for the first time, enables the use of a single test to reliably diagnose the presence or absence of tuberculosis infection in many different populations of animals across differing geographical areas and thus may form the basis of a universal test.

The invention relates to the use of specific polypeptides, antibodies, or nucleic acid molecules for the detection of TB disease or mycobacterial challenge. The use of a multi-peptide approach combined with the particular selection of highly immunogenic peptide fragments described herein facilitates the sensitive and specific diagnosis of TB.

Significantly, the specific combination of antigens used in the present invention allows diagnosis of tuberculosis infection across herds, geographical boundaries with levels of sensitivity specificity hitherto unobtainable across different populations of animals.

The polypeptides which may be used in the invention are SEQ ID NOs: 1-26 and variants and fragments thereof.

In one embodiment of the invention, the polypeptides which may be used in the invention comprise peptides having amino acid sequences comprising the amino acid sequences shown as one or more of SEQ ID NOs: 1-26.

SEQ ID NO: 4 corresponds to residues 1-34 of Rv3616c:
MSRAFIIDPTISAIDGLYDLLGIGIPNQGGILYS SEQ ID NO: 16 corresponds to Mb3905:
KWDATATELNNALQNL
SEQ ID NO: 17 corresponds to Mb3904(1):
KGSGSNIRQAGVQYSRADEEQQQ
SEQ ID NO: 18 corresponds to Mb3904(2):
KGSGSMAEMKTDAATLAQEAGN
SEQ ID NO: 19 corresponds to Mb2900:
KGSGSSVQGMSQDPVAVAASNNPEL
SEQ ID NO: 20 corresponds to Mb Mb2898:
KEKDKDGNGLVCGGVHTANATVYMIDTV
SEQ ID NO: 21 corresponds to Mb2002c:
DKGDGSAPKTYCEELKGT
SEQ ID NO: 22 corresponds to Mb1606c:
GVADNKWKRANCPVDVG
SEQ ID NO: 23 corresponds to PEP1 (MPB70):
KGSGSSVQGMSQDPVAVAASNNPEL
SEQ ID NO: 24 corresponds to PEP4 (CFP10):
KGSGSMAEMKTDAATLAQEAGN
SEQ ID NO: 25 corresponds to PEP9 (CFP10):
KGSGSNIRQAGVQYSRADEEQQQ
SEQ ID NO: 26 corresponds to PEP 17 (CFP10):
VVRFQEAANKQKQELDEI

Underlined sequences, as shown above for some peptides, were added to enhance the hydrophilicity of the peptides for binding to the surface. Where reference is made herein to use of such peptides, it should be understood that such peptides may be employed with or without the hydrophilicity enhancing sequence.

The inventors have found that the peptides having amino acid sequences SEQ ID NO: 1 to SEQ ID NO: 26 each elicit a strong immune response in serum from animals challenged with mycobacterial strains. Significantly, the inventors have found that by employing seven or more of the antigens, selected from the group consisting of an Rv3616 antigen, an MPB70 antigen, an MPB83 antigen, an ESAT 6 antigen, a CFP10 antigen, an α crystalline 2 antigen, a PE35 antigen, and a PPE68 antigen, the presence of tuberculosis may be identified in animals with a degree of sensitivity hitherto not obtainable.

Furthermore the inventors have also shown that a diagnostic test for tuberculosis infection demonstrating both high sensitivity and specificity may be based on only four antigens, namely an Rv3616 antigen, an MPB70 antigen, an MPB83 antigen, and an ESAT 6 antigen.

An MPB70 antigen may be the full length MPB70 protein, for example that having the amino acid sequence shown as Sequence ID No: 6. Alternatively, an MPB70 antigen may be a linear MPB70 epitope, for example the pep1(MPB70)antigen having the amino acid sequence shown as Sequence ID No: 23. In a particular embodiment of the invention, the test is based on an Rv3616 antigen, an MPB70 antigen, an MPB83 antigen, and an ESAT 6 antigen and a PEP1(MPB70) antigen. As described in the Examples, the sensitivity of the diagnostic test for diagnosing bovine tuberculosis increased from approximately 70% using a test based on determination of the presence of an immune response to Rv3616 antigen, an MPB70 antigen, an MPB83 antigen, and an ESAT 6 antigen to over 93% using a test based on the determination of the presence of an immune response to the same four antigens and to a pep1(MPB70)antigen. The marked improvement obtained when determining the presence of a response to the full length antigen and a response to the linear epitope was particularly unexpected.

Accordingly, in a first aspect of the invention, there is provided an assay method for the detection of Mycobacterium, in a biological sample, the method comprising the steps:
providing a biological sample from an animal; and
determining in vitro the presence or absence of, or the presence or absence of an immune response to, each member of the group of antigens comprising an Rv3616 antigen, a full length MPB70 protein antigen, an MPB70 linear epitope antigen, an MPB83 antigen, and an ESAT 6 antigen; wherein the identification of the presence of two or more of said antigens of said group of antigens, or of an immune response thereto, is indicative of the presence of Mycobacterium in the biological sample.

In one embodiment, the identification of the presence of two or more of said antigens of said group of antigens, or of an immune response thereto, is indicative of the presence of tuberculosis infection in the animal.

The peptides having amino acid sequences SEQ ID NO: 1 to SEQ ID NO: 26 each elicit strong immune responses in serum from animals infected with *Mycobacterium bovis* but are not present in either environmental strains of Mycobacterium or avian PPD strains.

Also described is an assay method for the detection of the presence of Mycobacterium, for example *Mycobacterium bovis,* in a biological sample, said method comprising the steps:
providing a biological sample; and determining the presence or absence of, or an immune response to, each member of a group of antigens in the biological sample, wherein said group of antigens comprises
   (i) the group consisting of an Rv3616 antigen, an MPB70 antigen, an MPB83 antigen, and an ESAT 6 antigen; or
   (ii) at least seven of the antigens of the group consisting of an Rv3616 antigen, an MPB70 antigen, and MPB83 antigen, an ESAT 6 antigen, a CFP10 antigen, an α crystalline 2 antigen, a PE35 antigen, and a PPE68 antigen;
   in the biological sample;
   wherein the identification of the presence of two or more of said antigens or of an immune response thereto is indicative of the presence of Mycobacterium in the biological sample.

In this method, the identification of the presence of one or more of said antigens or of an immune response thereto may be indicative of the presence of Mycobacterium in the biological sample.

Described herein is a method which involves determining the presence or absence of a group of antigens, or the presence or absence of an immune response to each member of said group of antigens, wherein said group of antigens comprises the group consisting of an Rv3616 antigen, an MPB70 antigen, an MPB83 antigen, and an ESAT 6 antigen, wherein the MPB70 antigen is a full length MPB70 protein. Also described is a method which involves determining the presence or absence of a group of antigens, or the presence or absence of an immune response to each member of said group of antigens, wherein said group of antigens comprises the group consisting of an Rv3616 antigen, an MPB70 antigen, an MPB83 antigen, and an ESAT 6 antigen, wherein the MPB70 antigen is an MPB70 linear epitope, for example PEP1 (MPB70). In a the first aspect of the invention, the method involves determining the presence or absence of a group of antigens, or the presence or absence of an immune response to each member of said group of antigens, wherein said group of antigens comprises both a full length MPB70 protein and an MPB70 linear epitope, for example PEP1 (MPB70).

In particular embodiments of the invention, the presence or absence of each of the antigens of the group consisting of Rv3616 antigen, an MPB70 antigen, and MPB83 antigen, an ESAT 6 antigen, a CFP10 antigen, an α crystalline 2 antigen, a PE35 antigen, and a PPE68 antigen is determined. As described in the examples, the use of these antigens greatly enhances the sensitivity of the test in identifying tuberculosis, providing unprecedented coverage across populations.

Unless the context demands otherwise (for example where reference is made to an MPB70 antigen and a pep1 (MPB70) antigen within the same context), reference to an MPB70 antigen should be understood to encompass an MPB70 antigen having the sequence shown as Sequence ID No: 6 or a pep1 (MPB70) antigen having the sequence shown as Sequence ID No: 23, or both. In one embodiment of the first aspect of the invention, the presence or absence of an immune response to both an MPB70 antigen having the sequence shown as Sequence ID No: 6 and a pep1 (MPB70) antigen having the sequence shown as Sequence ID No: 23 are determined. The combination of use of the MPB70 peptide together with the recombinant protein gives full coverage as this allows the identification of antibodies to both linear and conformational epitopes.

Likewise, unless the context demand otherwise, where reference is made to a CFP10 antigen, such reference should be understood to encompass one or more of a CFP10 antigen having the sequence shown as Sequence ID No: 9, a pep4 (CFP10) antigen having the sequence shown as Sequence ID No: 24, a pep9 (CFP10) antigen having the sequence shown as Sequence ID No: 25, or a pep17 (CFP10) antigen having the sequence shown as Sequence ID No: 26.

The present inventors have established that, even if an animal has been exposed to bovine PPD, the detectable immune response to the PPD test, in many cases, decreases rapidly after the initial exposure. Thus, although exposure to one or more of these antigens may be detectable in an animal uninfected with tuberculosis but which has been tested with bovine PPD within 2 weeks of the PPD test, within one month or so, the antigenic response will, in many cases, be undetectable. Thus, in general, unless a particular herd has been tested with PPD within one month preceding testing the animals with the method or kit of the present invention, the identification of the presence of an immune response to one or more of these antigens should be indicative of infection with *Mycobacterium bovis* and not merely exposure to bovine PPD.

However, the antigens having Sequence ID No: 9, Sequence ID No: 10 and Sequence ID No: 11 (rv1573, Rv1580c and Rv1585c) are not expressed in bovine PPD and thus may be used to confirm that the presence of a positive result is indicative of infection with bovine tuberculosis and not merely past exposure to PPD via e.g. the intradermal skin test.

Accordingly, in one embodiment of the first aspect of the invention, the method further includes determining the presence or absence of at least one of the antigens, or an immune response to at least one of the antigens, of the group consisting of an Rv1573 antigen, an Rv1580c antigen and an Rv1585c antigen. The identification of the presence of one or more of the Rv1573 antigen, the Rv1580c antigen and the Rv1585c antigen or of an immune response thereto is confirmatory of the presence of, or infection with, *Mycobacterium bovis.*

Where the selected polypeptide is present in the wild-type Mycobacterium but not in strains used for vaccines, such as Myobacterium bovis bacillus Calmette-Guerin (BCG), methods may be used to discriminate between infected animals and vaccinated animals. This will be particularly valuable in the control of tuberculosis in herds of animals.

Accordingly, also described herein is a method of determining whether an animal is infected with tuberculosis or vaccinated against tuberculosis, said method comprising the steps:
(a) providing a biological sample from said animal; and
(b) determining the presence or absence in the biological sample of:
   (i) at least three of the group consisting of an Rv3616 antigen, an MPB70 antigen, and MPB83 antigen, and an α crystalline 2 antigen, in the biological sample or an immune response thereto; and
   (ii) two or more of the group consisting of an ESAT6 antigen, a CFP10 antigen, a PE35 antigen, and
   a PPE68 antigen or an immune response thereto;
wherein the identification of the presence of (i) at least one of the group consisting of an Rv3616 antigen, an MPB70 antigen, and MPB83 antigen, and an α crystalline 2 antigen, or an immune response thereto, and (ii) at least one of the group consisting of an ESAT6 antigen, a CFP10 antigen, a PE35 antigen, and a PPE68 antigen, or an immune response thereto is indicative of the presence of infection of the animal with tuberculosis; and
wherein the identification of the presence of at least one of the group consisting of an Rv3616 antigen, an MPB70 antigen, and MPB83 antigen, and an α crystalline 2 antigen, or an immune response thereto, combined with the absence of each of the antigens tested of (ii), or an immune response thereto, is indicative of vaccination of the animal with a vaccine.

Of particular utility in this method is the identification of the presence of one or more ESAT6 antigen, a CFP10 antigen, a PE35 antigen, and a PPE68 antigen, or of an immune response to said antigens. These antigens are not present in BCG vaccines and thus detection of their presence, or of an immune response thereto, is indicative of a source of antigen, such as infection, other than such vaccination. Thus the presence of one or more of said antigens, or the presence of an immune response thereto, is indicative that the animal is infected with tuberculosis.

The inventors have determined that, by testing for presence of (i) seven or more of the group of antigens consisting of an Rv3616 antigen, an MPB70 antigen, and MPB83 antigen, an ESAT 6 antigen, a CFP10 antigen, an α crystalline 2 antigen, a PE35 antigen, and a PPE68 antigen, or (ii) the group of antigens consisting of an Rv3616 antigen, an MPB70 antigen, an MPB83 antigen, an ESAT 6 antigen, the sensitivity of testing for the presence of Mycobacteria and of tuberculosis infection is greatly improved.

The methods of the invention may be used with any animal, for example any mammal, including humans or bird. In one embodiment, the animal is a mammal. In one particular embodiment, the mammal is a non-human mammal.

In particular embodiment of the invention, the mammal is a cow.

A number of wild mammals are considered reservoirs for TB infection. For example, badgers are considered as a significant source of TB infection for cattle and , as a result, are often culled to prevent TB spread to cattle. However, this means of control of TB infection is controversial. A test which could reliably identify TB infection in badgers (and other host animals, which may act as reservoirs of infection) would therefore be invaluable in establishing whether or not individual animals, sets or populations thereof are indeed a threat to other species and thus save unnecessary culling. To date, however, a test which can reliably identify infection in animals such as badgers is not available. Typical tests have a reported sensitivity of less than 50% when assessing field samples (Chambers et al J Clin Microbiol. 2008 Apr;46(4):1498-500).

The methods of the present invention achieve considerably higher sensitivity than known tests for use in diagnosis of TB infection in animals such as badgers. Thus, in one embodiment of the invention, the mammal is a "reservoir" animal , for example a badger. In the context of the present invention, a "reservoir" animal is any non-human animal which may carry or be infected with tuberculosis and which may act as a source of infection of tuberculosis to domesticated livestock. Such reservoir animals include, in addition to badgers, white tail deer, possums, elephant and zoo/safari park animals.

Moreover, the inventors have determined that a considerable improvement in sensitivity may be achieved over known reservoir animals, e.g. badger, TB diagnostic assays by testing for the presence or absence of, or an immune response to, MPB70, Rv3616c and MPB83.

Accordingly, in a second aspect of the invention, there is provided a method of diagnosis of tuberculosis in a mammal, the method comprising the steps:
providing a biological sample from said mammal; and determining the presence or absence of, or the presence or absence of an immune response to, each member of the group of antigens comprising an Rv3616 antigen, a full length MPB70 protein antigen, an MPB70 linear epitope antigen, and an MPB83 antigen, wherein the identification of the presence of one or more of said antigens of said group of antigens, or of an immune response thereto, is indicative of the presence of tuberculosis infection in the mammal.

In one embodiment of the second aspect of the invention, the mammal is a "reservoir" mammal. In a particular embodiment, the mammal is a badger.

In the present application, unless the context demands otherwise, references to tuberculosis should be taken to refer to any tuberculosis, for example human tuberculosis, non-human animal tuberculosis, avian tuberculosis or a para-tuberculosis disease, such as Johne's disease.

Unless the context demands otherwise, reference to Mycobacterium should be taken to refer to any Mycobacterium.

In one embodiment, the Mycobacterium is Mycobacterium tuberculosis.

In another embodiment the Mycobacterium is *Mycobacterium bovis.*

In another embodiment, the Mycobacterium is *Mycobacterium avium.*

In another embodiment, the Mycobacterium is Mycobacterium paratuberculosis.

Any suitable biological sample may be used in the methods of the invention. Suitable biological samples include but are not limited to whole blood, serum, plasma, saliva, cerebrospinal fluid, urine and tissue samples.

In the methods of the invention, the presence of particular antigens or an immune response thereto may be determined using any means known in the art, either directly or indirectly. For example, in one embodiment, the presence of the antigen in the sample is determined; alternatively or additionally the presence of an antibody specific to said antigen is determined; alternatively, or additionally, the presence of an nucleic acid encoding said antibody or said antigen is determined.

The identification by the inventors of the particular selection of mycobacterial antigens which are particularly immunogenic, which are widely prevalent across geographical areas, and which moreover, cover each of the different stages of the TB life cycle enables the use of these polypeptides in the preparation of novel vaccines against tuberculosis. Such vaccines should provide a vaccinated animal with a sustained resistance to infection.

Accordingly, described herein is a vaccine or immunomodulator comprising at least seven of the antigens, or variants or fragments thereof, of the group consisting of an Rv3616 antigen, an MPB70 antigen, and MPB83 antigen, an ESAT 6 antigen, a CFP10 antigen, an α crystalline 2 antigen, a PE35 antigen, and a PPE68 antigen.

Such a vaccine may comprises an antigen (or variant or fragment thereof) of each member of the group consisting of an Rv3616 antigen, an MPB70 antigen, and MPB83 antigen, an ESAT 6 antigen, a CFP10 antigen, an α crystalline 2 antigen, a PE35 antigen, and a PPE68 antigen.

Given the demonstration of the high sensitivity of detection of TB infection in reservoir animals such as badgers when testing for the presence or absence of, or an immune response to, MPB70, Rv3616c and MPB83, it is believed that vaccines comprising at least these three antigens would be expected to have a significant effect on protecting against TB infection in such reservoir animals.

Thus, in a third aspect of the present invention, there is provided a vaccine for reservoir animals, said vaccine comprising an Rv3616 antigen, a full length MPB70 protein antigen, an MPB70 linear epitope antigen and an MPB83 antigen.

In one embodiment of the third aspect of the invention, the vaccine is a vaccine against tuberculosis.

In an alternative embodiment of the third aspect of the invention, one or more of said polypeptides are used in a vaccine as an adjuvant.

Also described is a method of providing immunity in an animal against tuberculosis, the method comprising administering to said animal a vaccine according to the third aspect of the invention.

Also described is the use of at least seven of the antigens, or variants or fragments thereof, of the group consisting of an Rv3616 antigen, an MPB70 antigen, and MPB83 antigen, an ESAT 6 antigen, a CFP10 antigen, an α crystalline 2 antigen, a PE35 antigen, and a PPE68 antigen in the preparation of a vaccine against tuberculosis.

In a fourth aspect of the invention, there is provided a diagnostic kit for the diagnosis of the presence of tuberculosis in a subject, wherein said kit:
(a) comprises an Rv3616 antigen, a full length MPB70 protein antigen, an MPB70 linear epitope antigen, an MPB83 antigen, and an ESAT 6 antigen; or
(b) comprises an antibody molecule with binding specificity for an Rv3616 antigen, an antibody molecule with binding specificity for a conformational epitope of a full length MPB70 protein antigen, an antibody molecule with binding specificity for an MPB70 linear epitope antigen, an antibody molecule with binding specificity for an MPB83 antigen, and an antibody molecule with binding specificity for an ESAT6 antigen; or
(c) is a kit for reservoir animals and comprises an Rv3616 antigen, a full length MPB70 protein antigen, an MPB70 linear epitope antigen, and an MPB83 antigen; or
(d) is a kit for reservoir animals and comprises an antibody molecule with binding specificity for an Rv3616 antigen, an antibody molecule with binding specificity for a conformational epitope of a full length MPB70 protein antigen, an antibody molecule with binding specificity for an MPB70 linear epitope antigen, and an antibody molecule with binding specificity for an MPB83 antigen.

Also described is a diagnostic kit for the diagnosis of the presence of tuberculosis in a subject, said kit comprising:
at least seven, such as eight, of the antigens (or variants or fragments thereof) of the group consisting of an Rv3616 antigen, an MPB70 antigen,
and MPB83 antigen, an ESAT 6 antigen, a CFP10 antigen, an α crystalline 2 antigen, a PE35 antigen, and a PPE68 antigen.

In one version of such a kit, the kit comprises each of the eight antigens (or variants or fragments thereof).

Also described is a diagnostic kit for the diagnosis of the presence of tuberculosis in a subject, said kit comprising an Rv3616 antigen, a MPB70 antigen, an MPB83 antigen, and an ESAT 6 antigen.

In one embodiment of the invention MPB70 linear epitope antigen is the PEP1 (MPB70) antigen. The kit comprises both a full length MPB70 protein and an MPB70 linear epitope.

In an embodiment of the fourth aspect of the invention, the kit further comprises at least one of the antigens selected from the group consisting of an Rv1573 antigen, an Rv1580c antigen or an Rv1585c antigen.

Also described herein is a diagnostic kit for the diagnosis of the presence of tuberculosis in a biological sample, said kit comprising:
at least seven of the group consisting of an antibody molecule with binding specificity for an Rv3616 antigen, an antibody molecule with binding specificity for an MPB70 antigen, an antibody molecule with binding specificity for an MPB83 antigen, an antibody molecule with binding specificity for an ESAT 6 antigen, an antibody molecule with binding specificity for a CFP10 antigen, an antibody molecule with binding specificity for an α crystalline 2 antigen, an antibody molecule with binding specificity for a PE35 antigen, and an antibody molecule with binding specificity for a PPE68 antigen.

In one version of such a kit, the kit comprises eight of the recited group of antibody molecules.

Also described is a diagnostic kit for the diagnosis of the presence of tuberculosis in a biological sample, said kit comprising: an antibody molecule with binding specificity for an Rv3616 antigen, an antibody molecule with binding specificity for an MPB70 antigen, and an antibody molecule with binding specificity for an MPB83 antigen.

In this kit, the MPB70 antigen may be a full length MPB70 protein. In an alternative, the MPB70 antigen is an MPB70 linear epitope, such as the PEP1 (MPB70) antigen. In a further alternative of this kit, the kit comprises both an antibody molecule with binding specificity for full length MPB70 protein and an antibody molecule with binding specificity for an MPB70 linear epitope.

The kit may further comprise at least one of the antibody molecules selected from the group consisting of an antibody molecule with binding specificity for an Rv1573 antigen, an antibody molecule with binding specificity for an Rv1580c antigen, and an antibody molecule with binding specificity for an Rv1585c antigen.

Also described is a diagnostic kit for the diagnosis of the presence of tuberculosis in a reservoir animal such as a badger, said kit comprising an Rv3616 antigen, an MPB70 antigen, and an MPB83 antigen.

Also described is a diagnostic kit for the diagnosis of the presence of tuberculosis in a biological sample from a reservoir animal such as a badger, said kit comprising: an antibody molecule with binding specificity for an Rv3616 antigen, an antibody molecule with binding specificity for an MPB70 antigen, and an antibody molecule with binding specificity for an MPB83 antigen.

Preferred features of each aspect of the invention are as for each of the other aspects *mutatis mutandis.*

### Detailed Description

### Polypeptides, Variants and Fragments

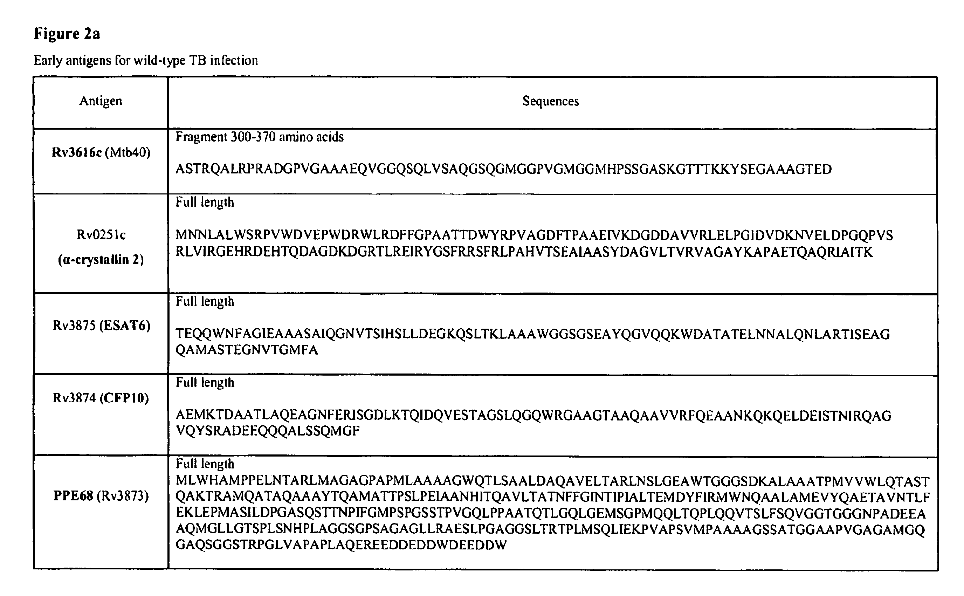

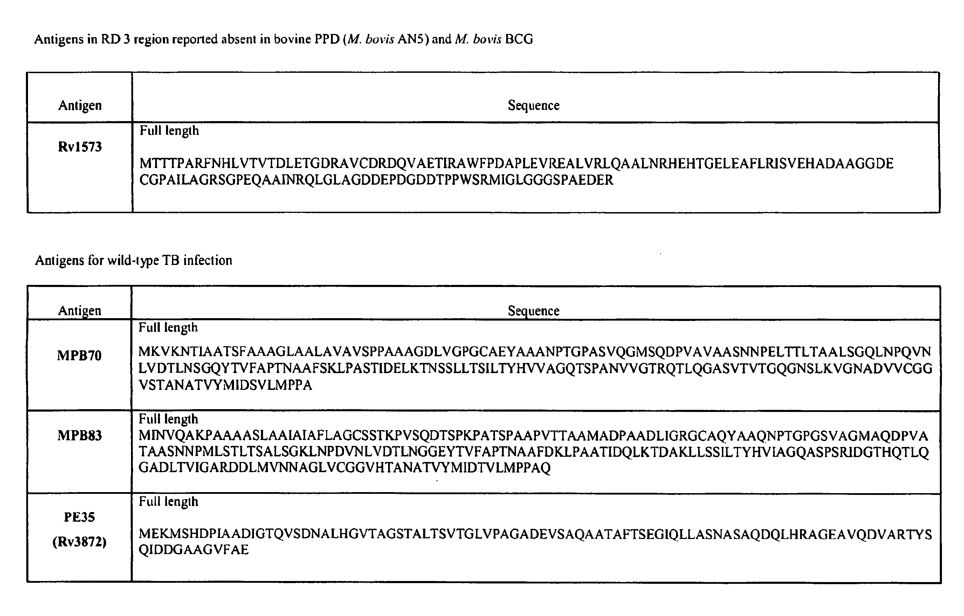

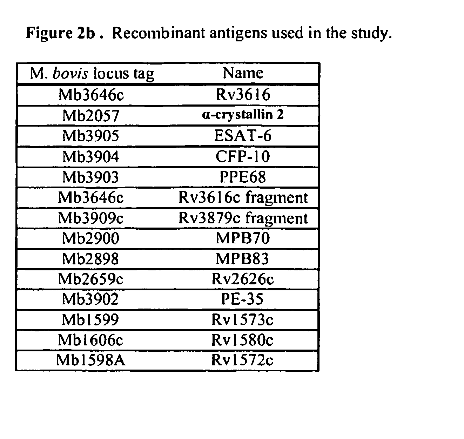

As described above, the inventors have identified a number of tuberculosis antigens which may be used in the diagnosis of tuberculosis, the differentiation between strains of Mycobacteria, and/or preparation of vaccines. The antigens comprise an Rv3616 antigen, an MPB70 antigen, and MPB83 antigen, an ESAT 6 antigen, a CFP10 antigen, an α crystalline 2 antigen, a PE35 antigen, and a PPE68 antigen. Polypeptides sequences for each of these antigens are provided in figure 2. In one embodiment of the invention the full length antigen sequences are used. However, fragments may also be used. For example, with respect to the rv3616c, examples of particular fragments which may be used are provided as SEQ ID Nos 2-5. In one embodiment of the invention, the antigens for use in the invention consist of polypeptides consisting of the amino acid sequence shown as any one of SEQ ID NO:1 to SEQ ID NO: 26.

However, the present invention is not limited to the use of polypeptides having such specific sequences of the polypeptides or antibodies disclosed herein but also extends to variants thereof. Thus, variant polypeptide sequences in which one or more amino acid residues are modified may also be used as the polypeptides in the invention. For example such variants may be useful in the preparation of vaccines or the raising of antibodies which may be used in kits of the invention. Modifications may involve insertion, addition, deletion and/or substitution of one or more amino acids. The modified amino acid residues in the amino acid sequences of the variant are preferably 30% or less, more preferably 20% or less, most preferably 10% or less, within the entire polypeptide. Such variants may be provided using the teaching of the present application and techniques known in the art. Preferably such variants involve the insertion, addition, deletion and/or substitution of 15 or fewer amino acids, more preferably of 10 or fewer, even more preferably of 5 or fewer, most preferably of 1 or 2 amino acids only.

Amino acid substitutions which do not essentially alter biological and immunological activities, have been described, e. g. by Neurath et al in"The Proteins"Academic Press New York (1979). Amino acid replacements between related amino acids or replacements which have occurred frequently in evolution are, inter alia,Ser/Ala,Ser/Gly, Asp/Gly, Asp/Asn,Ile/Val (see Dayhof, M. D. , Atlas of protein sequence and structure, Nat. Biomed. Res. Found. , Washington D. C., 1978, vol. 5, suppl. 3). Other amino acid substitutions may include but are not limited to Asp/Glu,Thr/Ser,Ala/Gly, Ala/Thr, Ser/Asn, Ala/Val, Thr/Phe, Ala/Pro, Lys/Arg,Leu/Ile, Leu/Val and Ala/Glu.

In preferred embodiments , a variant or fragment retains the immune reactivity of the polypeptide having the amino acid sequence shown as any one of SEQ ID NO:1 to SEQ ID NO: 26, of which it is a variant or fragment.

For the avoidance of any doubt, in the present application, where reference is made to the presence of two or more polypeptides selected from a list, for example two or more of the polypeptides selected from the polypeptides having the amino acid sequence shown as any one of SEQ ID NO:1, SEQ ID NO:2, SEQ ID NO:3, etc, it should be understood that reference is being made to at least one (first) polypeptide having one of the recited amino acid sequences and at least one (second) polypeptide having another of the recited amino acid sequences, which is different from the amino acid sequence of the first polypeptide.

Furthermore, for the avoidance of any doubt, in the present application, unless the context demands otherwise, where reference is made to the determination of the presence or absence of two or more polypeptides/antigens selected from a list, or the presence or absence of an immune response thereto, it should be understood that such a statement encompasses the determination of the presence or absence of two or more of said polypeptides/antigens selected from the list or the determination of the presence or absence of an immune response to two or more of said polypeptides/antigens or indeed a mixture thereof i.e. the determination of the presence or absence of one or more polypeptides/antigens selected from the list combined with the determination of an immune response to one or more other polypeptides/antigens selected from the list.

Furthermore for the avoidance of any doubt, where reference is made to MPB70 or variants thereof and pep1 (MPB70) together, pep1 (MPB70) should not be considered as an MPB70 variant.

### Antibody molecules

In the context of the present invention, an "antibody molecule" should be understood to refer to an immunoglobulin or part thereof or any polypeptide comprising a binding domain which is, or is homologous to, an antibody binding domain. Antibodies include but are not limited to polyclonal, monoclonal, monospecific, polyspecific antibodies and fragments thereof and chimeric antibodies comprising an immunoglobulin binding domain fused to another polypeptide.

Intact (whole) antibodies comprise an immunoglobulin molecule consisting of heavy chains and light chains, each of which carries a variable region designated VH and VL, respectively. The variable region consists of three complementarity determining regions (CDRs, also known as hypervariable regions) and four framework regions (FR) or scaffolds. The CDR forms a complementary steric structure with the antigen molecule and determines the specificity of the antibody.

Fragments of antibodies may retain the binding ability of the intact antibody and may be used in place of the intact antibody. Accordingly, for the purposes of the present invention, unless the context demands otherwise, the term "antibodies" should be understood to encompass antibody fragments. Examples of antibody fragments include Fab, Fab', F(ab')2, Fd, dAb, and Fv fragments, scFvs, bispecific scFvs, diabodies, linear antibodies (see US patent 5,641,870, Example 2 ; Zapata et al., Protein Eng 8 (10): 1057-1062 [1995]); single-chain antibody molecules; and multispecific antibodies formed from antibody fragments.

The Fab fragment consists of an entire L chain (VL and CL), together with VH and CH1. Fab' fragments differ from Fab fragments by having an additional few residues at the carboxy terminus of the CH1 domain including one or more cysteines from the antibody hinge region. The F(ab')2 fragment comprises two disulfide linked Fab fragments.

Fd fragments consist of the VH and CH1 domains.

Fv fragments consist of the VL and VH domains of a single antibody.

Single-chain Fv (scFv) fragments are antibody fragments that comprise the VH and VL domains connected by a linker which enables the scFv to form an antigen binding site (see Pluckthun in The Pharmacology of Monoclonal Antibodies, vol.113, Rosenburg and Moore eds., Springer-Verlag, New York, pp. 269-315 (1994).

Diabodies are small antibody fragments prepared by constructing scFv fragments with short linkers (about 5-10 residues) between the VH and VL domains such that inter-chain but not intra-chain pairing of the V domains is achieved, resulting in a multivalent fragment, i.e. a fragment having two antigen-binding sites (see, for example, EP 404 097; WO 93/11161; and Hollinger et al., PNAS. USA, 90: 6444-6448 (1993)).

Further encompassed by fragments are individual CDRs. The CDRs may be carried on a framework structure comprising an antibody heavy or light chain sequence or part thereof. Preferably such CDRs are positioned in a location corresponding to the position of the CDR(s) of naturally occurring VH and VL domains. The positions of such CDRs may be determined as described in Kabat et al, Sequences of Proteins of Immunological Interest, US Dept of Health and Human Services, Public Health Service, Nat'l Inst. of Health, NIH Publication No. 91-3242, 1991 and online at www.kabatdatabase.com http://immuno.bme.nwu.edu. Furthermore, modifications may alternatively or additionally be made to the Framework Regions of the variable regions. Such changes in the framework regions may improve stability and reduce immunogenicity of the antibody.

The antibody molecules for use in the present invention extend, for example, to any other antibody which specifically binds a polypeptide identified herein as inducing a strong immune response i.e. an antibody molecule which retains binding specificity for a polypeptide consisting of amino acid sequence shown as any one of SEQ ID NOs:1 to 26.

Antibodies for use in the invention herein include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see US 4,816,567; and Morrison et al., PNAS. USA, 81: 6851-6855 (1984)). Chimeric antibodies of interest herein include "primatized"antibodies comprising variable domain antigen-binding sequences derived from a non-human primate(e.g. Old World Monkey, Ape etc), and human constant region sequences.

Antibody molecules for use in the present invention may be produced in any suitable way, either naturally or synthetically. Such methods may include, for example, traditional hybridoma techniques (Kohler and Milstein (1975) Nature, 256 :495-499), recombinant DNA techniques (see e.g. US 4,816, 567), or phage display techniques using antibody libraries (see e.g. Clackson et al. (1991) Nature, 352: 624-628 and Marks et al. (1992) Bio/ Technology, 10: 779-783). Other antibody production techniques are described in Antibodies: A Laboratory Manual, eds. Harlow et al., Cold Spring Harbor Laboratory, 1988.

Traditional hybridoma techniques typically involve the immunisation of a mouse or other animal with an antigen in order to elicit production of lymphocytes capable of binding the antigen. The lymphocytes are isolated and fused with a myeloma cell line to form hybridoma cells which are then cultured in conditions which inhibit the growth of the parental myeloma cells but allow growth of the antibody producing cells. The hybridoma may be amenable to genetic mutation, which may or may not alter the binding specificity of antibodies produced. Synthetic antibodies can be made using techniques known in the art (see, for example, Knappik et al, J. Mol. Biol. (2000) 296, 57-86 and Krebs et al, J. Immunol. Meth. (2001) 2154 67-84).

Modifications may be made in the VH, VL or CDRs of the antibody molecules, or indeed in the FRs using any suitable technique known in the art. For example, variable VH and/or VL domains may be produced by introducing a CDR, e.g. CDR3 into a VH or VL domain lacking such a CDR. Marks et al. (1992) Bio/ Technology, 10: 779-783 describe a shuffling technique in which a repertoire of VH variable domains lacking CDR3 is generated and is then combined with a CDR3 of a particular antibody to produce novel VH regions. Using analogous techniques, novel VH and VL domains comprising CDR derived sequences of the present invention may be produced.

Alternative techniques of producing antibodies for use in the invention may involve random mutagenesis of gene(s) encoding the VH or VL domain using, for example, error prone PCR (see Gram et al, 1992, PNAS 89 3576-3580. Additionally or alternatively, CDRs may be targeted for mutagenesis e.g. using the molecular evolution approaches described by Barbas et al 1991 PNAS 3809-3813 and Scier 1996 J Mol Biol 263 551-567.

Having produced such variants, antibodies and fragments they may be tested for binding to *Mycobacterium bovis.*

The antibodies for use in the invention may comprise further modifications. For example the antibodies can be glycosylated, pegylated, or linked to albumin or a nonproteinaceous polymer.

Antibodies for use in the invention may be labelled. Labels which may be used include radiolabels, enzyme labels such as horseradish peroxidase or alkaline phosphatase, or biotin.

### Nucleic Acid

Nucleic acid for use in the present invention may comprise DNA or RNA. It may be produced recombinantly, synthetically, or by any means available to those in the art, including cloning using standard techniques.

The nucleic acid may be inserted into any appropriate vector. The vector may be an expression vector and the nucleic acid may be operably linked to a control sequence which is capable of providing expression of the nucleic acid in a host cell. A variety of vectors may be used. For example, suitable vectors may include viruses (e. g. vaccinia virus, adenovirus,etc., baculovirus); yeast vectors, phage, chromosomes, artificial chromosomes, plasmids, or cosmid DNA.

The vectors may be used to introduce the nucleic acids into a host cell. A wide variety of host cells may be used for expression of the nucleic acid of the invention. Suitable host cells for use in the invention may be prokaryotic or eukaryotic. They include bacteria, e.g. E. coli, yeat, insect cells and mammalian cells. Mammalian cell lines which may be used include Chinese hamster ovary cells, baby hamster kidney cells, NSO mouse melanoma cells, monkey and human cell lines and derivatives thereof and many others.

A host cell strain that modulates the expression of, modifies, and/or specifically processes the gene product may be used. Such processing may involve glycosylation, ubiquiination, disulfide bond formation and general post-translational modification.

For further details relating to known techniques and protocols for manipulation of nucleic acid, for example, in preparation of nucleic acid constructs, mutagenesis, sequencing, introduction of DNA into cells and gene expression, and analysis of proteins, see, for example, Current Protocols in Molecular Biology, 5th ed., Ausubel et al. eds., John Wiley & Sons, 2005 and, Molecular Cloning: a Laboratory Manual: 3rd edition Sambrook et al., Cold Spring Harbor Laboratory Press, 2001.

### Diagnostic Methods, Assays and Kits

The invention may be used in the diagnosis of a variety of conditions and disorders associated with tuberculosis. These include *Mycobacterium bovis, Mycobacterium avium* and human mycobacterium and para-tuberculosis diseases such as Johne's disease.

In using the methods of the invention to identify the infection with a mycobacterial strain, either as current or previous infection, the presence or absence of immunogenic polypeptides, or the presence or absence of an immune response to said polypeptides is determined from a biological sample. Any suitable biological sample may be used. For example, the biological sample may be a biological fluid, such as sputum, saliva, plasma, blood, urine or sperm, or a tissue, such as a biopsy of a tissue.

Diagnostic and assay means of detecting the presence of polypeptides or an immune responses to said polypeptides are known in the art. For example, the presence of the polypeptides may be detected by use of antibodies specific to said polypeptides.

Alternatively, using standard techniques in the art, the presence of nucleic acids encoding the polypeptide or indeed an antibody specific to said polypeptide may be used. Further, the presence of antibodies specific to said polypeptides may be used to determine the presence of an immune response to said polypeptide.

Techniques which may be employed include but are not limited to ELISA, Immunohistochemistry, Electron Microscopy, Latex agglutination, Immuno Blotting, immunochromatography, immunochips, lateral flow immunoassays and Dip Stick Immuno testing.

The ELISA test (enzyme linked immunoenzymatic assay) is frequently used for serological diagnosis. This method allows the identification and quantification of antigens or antibodies in biological fluids. The conventional ELISA consists in the detection of the complex antibody-antigen by a second antibody (against the antibody that reacts with the antigen) conjugated to an enzymatic activity (peroxidase, alkaline phosphatase and others).

In the latex agglutination assay, the antigen preparation is affixed to latex beads. The biological sample is then incubated directly on a slide with the latex particles. In a short time the reaction is examined for the presence of crosslinked, or agglutinated latex particles indicating the presence of antibodies to polypeptides in the sample.

Immunochips may used to determine the presence of the specific Mycobacterium antigens. Generally, the specific antibodies to the antigens are immobilised on a transducer, e.g. electrodes, caloric meter, piezoelectric crystal, surface plasmon resonance transducer, surface acoustic resonance transducer or other light detecting device. The binding of antigens in the biological sample to the immobilised specific antibody is detected by a change in electric signal.

As described above, the presence of the immunogenic antigens may be detected by detecting nucleic acids encoding the antigen or encoding antibodies raised against the antigen. Such techniques are well known in the art. For example, where large amounts of DNA are available, genomic DNA may be used directly. Alternatively, the region of interest is cloned into a suitable vector and grown in sufficient quantity for analysis. The nucleic acid may be amplified by conventional techniques, such as the polymerase chain reaction (PCR) (Saiki, et al. (1985) Science 239:487). Primers may be used to amplify sequences encoding the polypeptide of interest. Optionally, a detectable label, for example a fluorochrome, biotin or a radioactive label may be used in such an amplification reaction. The label may be conjugated to one or both of the primers. Alternatively, the pool of nucleotides used in the amplification is labeled, so as to incorporate the label into the amplification product.

The sample nucleic acid, e.g. amplified or cloned may be analysed using any suitable method known in the art. For example, the nucleic acid may be sequenced by dideoxy or other methods, and the sequence of bases compared to the deleted sequence. Hybridization with the variant sequence may also be used to determine its presence, by Southern blots, dot blots, etc. The hybridization pattern of a control and variant sequence to an array of oligonucleotide probes immobilized on a solid support, as described in WO95/35505, may be used as a means of detecting the presence or absence of a sequence.

In one embodiment, the kit contains an antigen preparation prepared as described above and then fixed onto a solid support for use in a serological assay. The kit may also contain an explanatory note on how to proceed.

In one embodiment, the assay is in a dip stick or test strip format. Such assays typically employ a solid support medium, which is able to bind target substances such as proteins, for example protein antigens. In use, the test strip is exposed to a sample, for example serum, such that antibodies in the serum may bind target substances such as antigens on the test strip. After washing, the dip stick/test strip is exposed to a labelled probe which binds the bound antibody of interest, with binding typically visualised by means of further incubation with a visualisable substrate, for example a chromogenic substrate.

In a further embodiment, the assay of the invention is provided as a lateral flow immunoassay. Such devices are well known in the art. Lateral flow immunoassay devices typically employ a microporous element along which a sample may flow laterally, the device having a capture region for binding an analyte of interest in the sample. Generally, the lateral flow device has an application zone, to which a sample is applied, a conjugate zone, containing a mobile reporter conjugate, which is typically a visually observable reporter e.g. colloidal gold conjugated to an antibody directed against the analyte and a capture region comprising a capture agent which binds the analyte. In use, the sample is applied to the application zone and flows along a strip to a first region, at which the reporter conjugate binds the analyte. Thereafter, movement of the reporter conjugate/analyte complex to the capture region results in capture of the complex at that region. Uncomplexed reporter conjugate does not bind at the capture region. By determining the amount of visually detectable signal at the capture region, the presence of analyte in a sample may be determined. Variants of such kits are well known in the art.

In one embodiment of the invention, the methods, assays and kits of the invention comprise or use an assay in which the presence or absence of a number of antigens or n immune response thereto is analysed simultaneously, i.e. using a multiplex based assay. Any suitable multiplex assay may be used. For example, arrays of different tuberculosis antigens may be provided in microplate wells and a sample mixed with reporter antibodies in the wells. Other multiplex based methods may involve protein arrays placed on a matrix, with the response to individual proteins on the solid-phase array assayed. The use of such multiplex techniques enables the rapid identification of the presence or absence of a plurality of target antigens (or antibodies) during a single run of the analysis.

As described in the Examples, the inventors have developed a multiplex technique in which a plurality of different antigens, for example up to 25, are spotted in a single well of a 96 well plate. Surprisingly, the inventors have shown that high sensitivity of reaction with each of the antigens tested was possible in the multiplex reactant mixture despite the necessary requirement of a single buffer being used for each of the plurality of antigen- antibody reactions in a single well.

Using such a plate, up to 96 samples, each from a different animal, may be tested on a single plate with the each samples reactivity with each of the antigens measured simultaneously. Such a technique therefore enables the rapid analysis of infection with, for example, tuberculosis, in a large number of animals simultaneously.

Moreover, the inventors have surprisingly found that the advantages of such a system for the determination of tuberculosis infection is not just with respect to the convenience of being able to test many different antigens in many different samples simultaneously. To their surprise, the inventors have also demonstrated that the multiplex platform provided higher sensitivity and specificity than that obtained using conventional assays such as lateral flow assays and ELISA.

Accordingly, also described herein is a multiplex method of diagnosis of tuberculosis in an animal, the method comprising the steps:
providing a substrate on which is immobilised a plurality of different antigens,
bringing into contact with said substrate a biological sample from an animal,
identifying the presence or absence of binding of antibodies from said sample with said antigens, wherein the determination of binding is indicative of the presence of tuberculosis infection in the animal.

The substrate may be a microplate. In one such method, a plurality of different antigens are immobilised in a single well of a plate. In one version of this method, the plurality of antigens comprise two or more, for example more than 5, 10, 15 or 20 of the antigens having amino acid sequences SEQ ID NO: 1 to SEQ ID NO: 26, or fragments or variants thereof.

Also described is a multiplex method of diagnosis of tuberculosis in an animal, the method comprising the steps:
providing a substrate to which is attached a plurality of different antibodies,
bringing into contact with said substrate a biological sample from an animal,
identifying the presence or absence of binding of antigens from said sample with said antibodies, wherein the determination of binding is indicative of the presence of tuberculosis infection in the animal.

The substrate may be a microplate. In one such method, a plurality of different antibodies are immobilised in a single well of a plate. In one version of this method, the plurality of antibodies comprises two or more, for example more than 5, 10, 15 or 20 antibodies each having binding specificity for a different member of the group of antigens having amino acid sequences Sequence ID No: 1 to Sequence ID No: 26.

The kit can be used to perform the methods of the invention described above.

### Vaccines

As described above, the present invention also extends to vaccines for use in protecting against tuberculosis and tuberculosis-associated diseases.

One way of making a vaccine according of or for use in the invention is by biochemical purification of the immunogenic polypeptides from bacteria.

Alternatively, expression products of the genes encoding the polypeptides according to the invention may be used in vaccines. Such vaccines based upon the expression products of these genes can easily be made by admixing one or more proteins with a pharmaceutically acceptable carrier.

Alternatively, a vaccine according to the invention can comprise live recombinant carriers, capable of expressing the polypeptides according to the invention.

Vaccines described above all contribute to active vaccination, i.e. the host's immune system is triggered by one or more proteins according to the invention or immunogenic fragments thereof, to make antibodies against these proteins.

Alternatively, such antibodies can be raised in e.g. rabbits or can be obtained from antibody-producing cell lines. Such antibodies can then be administered to the host animal. This method of vaccination, passive vaccination, is the vaccination of choice when an animal is already infected, and there is no time to allow the natural immune response to be triggered. It is also the preferred method for vaccinating immune-compromised animals.

Therefore, also described are vaccines comprising antibodies against one or more of the immunogenic polypeptides used in the invention.

An alternative and efficient way of vaccination is direct vaccination with DNA encoding the relevant antigen. Direct vaccination with DNA encoding proteins has been successful for many different proteins. (As reviewed in e. g. Donnelly et al., The Immunologist 2:20-26(1993)). Therefore, also described are vaccines comprising nucleic acid sequences encoding a polypeptide as used in the invention or immunogenic fragments thereof, and vaccines comprising DNA fragments that comprise such nucleic acid sequences.

Still other forms of this vaccines comprise recombinant DNA molecules.

DNA vaccines can easily be administered through intradermal application e. g. using a needle-less injector.

Vaccines according to the present invention may comprise a pharmaceutically acceptable carrier e. g. sterile water, a sterile physiological salt solution, or a buffer, and may also contain an adjuvant.

The invention will now be described further in the following non-limiting examples. Reference is made to the accompanying drawings in which:
Figure 1 shows a Venn diagram illustrating the expression of antigens between different Mycobacterium strains; Group 1 (strain differentiation) - Rv1573, RV1580c, Rv1585c, Rv1572c; Group 2 (Early antigen) - Rv3616c, ESAT6, CFP10, alpha-crystallin 2, PPE68; Group 3 (covering complete life cycle)- MPB70, MPB83,and PE35.
Figure 2(a) shows the amino acid sequences of antigens used in the present invention.
Figure 2(b) shows recombinant antigens used in the multiplex assay.
Figure 3 shows an example of the captured image of the test wells from the multiplex ELISA for one of the animals from the infectivity study. Each well shown represents a sample point for animal A (pre-challenge, 2, 5, 10 and 17 weeks post challenge).
Figure 4 illustrates an image of 96 well plate from the multiplex assay. Negative and positive controls are located in wells 1 A-E. This plate shows a representative group of TB positive and negative animal samples screened against 11 antigens.

### Example 1

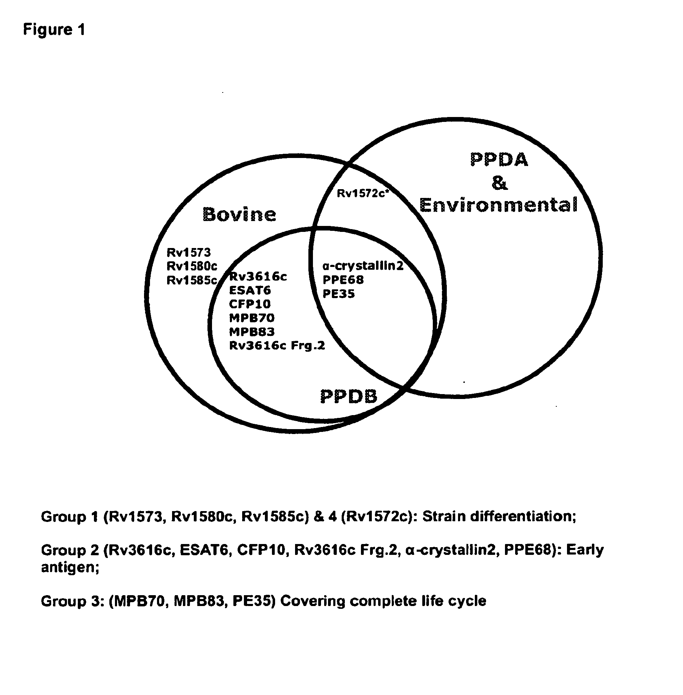

The inventors have used bioinformatics software to identify and characterise proteins which are specific to one or other mycobacterial strains. Using a genome alignment strategy the group has identified point mutations or deletions which allow differentiation between *M bovis,* PPD *(M. bovis* AN5 and *M. avium), M. bovis* BCG and environmental mycobacteria (Figure 1). They have identified a panel of proteins capable of strain identification (Figure 2 (a)).

As described below, the inventors have surprisingly found that by using a panel of seven or eight of these antigens, compared to known techniques, the accuracy of detection of tuberculosis may be greatly improved.

Significantly, a high degree of sensitivity demonstrated using this particular panel of antigens is obtainable across cohorts of tested animals from different geographical regions, giving a level of coverage which is unprecedented in tuberculosis testing.

### Methodology

### Cloning, expression and purification of mycobacterial antigens

DNA encoding target mycobacterial antigens (Figure 2(a)) were identified and analysed by bioinformatics software. Primers were designed to amplify selected antigenic fragments of each of the target antigen by PCR. The PCR mixture contained 10 ng *M. tuberculosis* genomic DNA as template in a total volume of 50 µl with 47 µl *Taq* polymerase supermix (1 Unit of recombinant enzyme), and 1µl of each primer (10 pMol). The amplification was carried out with initial heating at 95 °C for 4 min followed by 25 cycles of denaturation at 95 °C for 30 s, annealing at 55 °C for 30 s, and extension at 72 °C for 1 min in each cycle. The final polymerisation was carried out for 10 min. The PCR products were analysed by 1.5% agarose gel electrophoresis, and purified. Each PCR product after purification was digested with appropriate enzymes and cloned in respective FET expression vectors with C-terminal (His6) tag. The resultant constructs were characterised by sequencing using Big dye terminator chemistry and automated DNA sequencer (ABI Prism 3100).

Recombinant mycobacterial antigens were expressed by FET system. Competent *E.coli* cells were transformed with various recombinant plasmids. The successful transformed cells were picked and grown into 5 ml LB containing 100 µg/ml of ampicillin at 37 °C with shaking overnight. 100 µl of this culture was used to inoculate 500 ml of pre-warmed medium with antibiotics listed above and cultured at 37 °C for 120 min at 300 rpm. Expression of recombinant antigen was induced from the cells at this time with 1µl / ml of 25mM IPTG, and the cells cultured for a further 4 hours. The cells were then pelleted by centrifugation for 15 min and the pellets were resuspended in 100 ml 8 M urea and lysed overnight at room temperature with shaking. The lysates were analysed by SDS-PAGE for presence of recombinant mycobacterial antigens.

Recombinant mycobacterial antigens were purified by using FET purification system. Antigen fragments were purified from the lysates using affinity chromatography. The elution was monitored at 280 nm and 2 ml fractions were collected. The fractions were analysed by SDS-PAGE followed by Commassie/Sliver staining and Western Blot.

### ELISA assays

### Part 1: Plate Coating

1. The plate frames were labelled with protein name and date of coating
2. The plates were coated, at the relevant protein concentration (the protein is diluted in the coating buffer), by adding 100µl/well.
3. The plates were covered with a plate sealer and incubated at 2-8°C on a level surface overnight.
4. The plates were washed with EW2 (1X) and tapped dry on tissue paper

### Part 2: Plate Blocking

5. The plates were blocked by adding 200µl of the Blocking Buffer to each well.
6. The plates were covered with a plate sealer.
7. The plates were incubated for 30 minutes at 37°C Stationary.
8. The plates were washed with EW2 and tapped dry on tissue paper.

### Part 3: The Assay

9. The sample was diluted to a 1:250 dilution in the Sample Dilution Buffer). Sample dilutions were carried out on the Tecan Genesis automated system or manually.
10. Diluted samples were transferred to the test plates on the Tecan Genesis or manually - 100µl of sample is added to each well of the test plate according to the plate layout required.
11. The plate was covered with a plate sealer and incubated at 34°C Shake Speed 3 for 30min.
12. The plate was washed with EW2 and tapped dry on tissue paper.
13. The conjugate was prepared to a 1:2500 dilution in the conjugate dilution buffer
14. 100µl of conjugate was added to each well on the Tecan Genesis or manually.
15. The plate was covered with a plate sealer and incubated at 34°C Shake Speed 3 for 1 hour.
16. The plate was washed with EW2 and tapped on tissue paper.
17. TMB was prepared by adding equal volumes of TMB Concentrate to TMB Diluent.
18. 100µl of TMB was added to each well.
19. The plate was covered with a plate sealer and incubated at 34°C Shake Speed 3 for 15 mins.
20. 100µl of 0.5M H₂SO₄ Stop Solution was added to each well to stop the reaction.
21. The plate was read at 450-690nm and the results saved. The settings for the Tecan sunrise reader settings were: absorbance, 450nm - 690nm, shake low intensity outside 10 sec. no settle time.

### Buffer Preparation

| **Sodium Hydrogen Carbonate Solution** | |
|---|---|
| Sodium Hydrogen | 4.2g |
| Carbonate (NaHCO₃) | |

| Deionised Water | 1000ml |
|---|---|
| **Sodium Carbonate Solution** | |
| Sodium Carbonate (Na₂CO₃) | 5.2g |

| Deionised Water | 1000ml |
|---|---|
| **Coating Buffer** | |
| Sodium Hydrogen | 1000ml |
| Carbonate (NaHCO₃) | |
| Solution | |
| Sodium Carbonate | until pH 9.5 is achieved |
| Solution (Na₂CO₃) | |

| **PBS Buffer** | |
|---|---|
| PBS Tablet | 1 tablet |
| Deionised Water | 200ml |

| **Blocking Buffer** | |
|---|---|
| PBS | 1000ml |
| DGS (1+10) | 100ml |

| **Sample Dilution Buffer** | |
|---|---|
| 1% Casein | 720ml (made in PBS) |
| DGS (1+10) | 72ml |

| **Conjugate Dilution Buffer** | |
|---|---|
| EW2 (1X) | 100ml |
| DGS (1+50) | 2ml |

### Results

The inventors grouped the antigens into three different categories and each category has been specified with unique strains differentiation or early bovine TB detection ability (Fig. 1). The 1^{st} group comprises RD3 deletion (Rv1573) which can differentiate between *M. bovis* wild type and bovine PPD. The 2^{nd} group comprises early antigens (Rv3616c, ESAT6, CFP10 & α-crystallin 2) which have the ability to detect early bovine TB infection. The 3^{rd} group (MPB70, MPB83 & PE35) comprises antigens covering the complete life-cycle of *M. bovis* wild type.

### Expression and purification of proteins

Transformed cells harbouring the correct constructs for target proteins (the sequences of which are shown in Figure 2(a)) were selected by colony PCR and sequencing. Expressed target proteins were identified by SDS PAGE analysis of cell lysates (Data not shown). Each target protein was successfully purified and polished by IMAC following IPTG induced expression and endotoxin removal resins respectively. Protein purity & endotoxin level (Quality Control) was confirmed using SDS-PAGE Commassie blue stain, Sliver stain, Western blot analysis (E.C.L./DAB) and endochrome kit.

### ELISA assays

### Sensitivity Summary

| **Samples** | **No.** | **Proteins used** | **Samples detected** | **%** |
|---|---|---|---|---|
| *Study 1* | 291 | MPB70 only | 203/291 | 69.7 |
| *Study 2* | 114 | 7 Proteins | 94/114 | 82.5 |
| *Study 3* | 61 | 8 Proteins | 59/61 | 96.7 |

| | | | | |
|---|---|---|---|---|
| 7 Proteins Panel = MPB70, MPB83, Rv3616c, ESAT6, CFP10, a-crystallin2, PE35 8 Proteins Panel = MPB70, MPB83, Rv3616c, ESAT6, CFP10, a-crystallin2, PE35 and PPE68. | | | | |

In addition, the samples employed in study 3 were analysed using only MPB83, ESAT-6 and CFP-10 antigens only and compared with the results with the same cohort of animals as tested with the eight protein panel as described above. It was found that, using MPB83, ESAT-6 and CFP-10 only, the maximum sensitivity obtained in identifying tuberculosis was 86.9%. As noted above, with all eight antigens, the sensitivity was increased to 96.7%, representing a highly significant improvement in sensitivity when employing the panel of the eight specific antigens of the invention.

### Example 2 Multiplex Assays

### Methods

### Serum samples

Serum samples used in the study were obtained from several sources. Blood samples were taken into serum tubes (Vacuette, serum clot activator tubes, Greiner-bio-one), transported at room temperature and then stored at 2-8 °C until processing. Following centrifugation (3000g, 30 minutes at 2-8°C) the serum was removed, aliquoted and stored at -20°C.

Two sets of sera were obtained, one from a negative sample bank including sera from herds of animals with a known history of being free of *Mbv.* A third group of sera were collected from animals that were proven to be positive for *Mbv* infection at the time of slaughter based on subsequent histopathological/bacteriological examination.

The fourth set of serum samples was part of an infectivity study. The animals were non-vaccinated and challenged via the intra-tracheal route with a low dose of a virulent strain of *Mbv* (approximately 5,000 CFU). Sera were collected prior to challenge and then at 2, 5, 10 and 17 weeks post infection (PI) (data not shown). A single intradermal comparative cervical tuberculin test (SICCT) was carried out prior to challenge and also during week 15 PI. All animals in the study had lesions typical of a TB infection, and all animals at 17 weeks PI were culture positive for *Mbv.*

### Preparation of antigens

The genes encoding different TB proteins were expressed in *E. coli* as N-terminal polyhistidine-tagged (6X HIS) fusion proteins by Fusion Antibodies Ltd. (Belfast) using the Fusion Expression Technology (FET) platform. Recombinant proteins were purified and polished to near homogeneity by using Fusion Antibodies Ltd. three-step chromatographic protocol. SDS-PAGE and western blot analysis were performed on all purified and polished recombinant TB proteins to confirm their level of purity was greater than 99%. Synthetic peptides were synthesised by Genosphere Biotechnologies (France) to a purity of >80% (RP-HPLC at 220nm). Amino acid residues were added to peptides (see Figure 2(b) (ii) to enhance the hydrophilicity of the peptides. Lyophilised peptides were re-constituted to 2mg/ml in sterile phosphate buffer saline pH 7.4 (Sigma Aldrich, Dublin) and aliquots were prepared and stored at -20°C. Antigen quantification was performed using the micro BCA™ protein assay kit (Pierce, Rockford, IL). See Figures 2b (i) and 2b(ii) for the list of antigens (recombinant proteins and synthetic peptides) used in this study.

### Multiplex Antigen printing

Antigens used in the study were printed in each well of a black polystyrene 96 well plate in a multiplex planar array format by Quansys Biosciences, (Logan, UT). Optimization of the antigen printing was carried out in conjunction with Enfer. Plates were printed by Quansys Biosciences, (Logan, UT) and testing of plates was carried out at Enfer Scientific (Naas, Ireland). Buffer mediated alterations in spot deposition and morphology were examined at Quansys Biosciences using an Alpha Innotech 8900 imager workstation and a Nikon Diaphot ELWD phase contrast inverted microscope. The effects of differing buffers used for spotting were screened for differential responses at Enfer. The buffer selected gave the optimal pixel intensity response with the optimal spot size/morphology on the plates. Antigen coating concentrations optimization was carried out at Enfer Scientific (Naas, Ireland) using a series of coating titrations printed by Quansys Biosciences in the optimized printing buffer. Panels of antigens were printed using the optimized printing protocol and buffers and were used for sample testing. All plates were shipped and stored at 2-8°C. Plates were allowed to warm to room temperature for 30 minutes prior to use.

### Multiplex immunoassay method

The assay method was developed in-house at Enfer Scientific (Naas, Ireland). Serum samples were diluted 1:250 into sample dilution buffer (Enfer Buffer A, Enfer Scientific, Naas, Ireland) and mixed. 30µl of sample was added per well. Sample dilution and plating was carried out using the automated pipettor Tecan Genesis RSP 150. The plates were incubated at room temperature with agitation (900 rpm) for 30minutes. The plates were washed with 1X Enfer Wash Buffer (Enfer Scientific, Ireland) six times and aspirated. The detection antibody (polyclonal rabbit anti-bovine immunoglobulin -HRP, Dako, Denmark) was prepared to a dilution of 1:3000 in detection antibody dilution buffer (Enfer Buffer B, Enfer Scientific, Naas, Ireland). After addition of 30µl of the detection antibody to test wells, the plates were incubated at room temperature for 15 minutes with agitation (900 rpm). The plates were washed as above and 30µl of substrate (50:50 dilution of substrate and diluent) added per well (Chemilumenscent substrate and diluent, Quansys Biosciences, Logan, Utah). Signals were captured over a 45 second exposure time using Quansys Biosciences Imaging system (Quansys Biosciences, Logan, Utah). Images were saved as CR2 image files. Data were extracted from the captured images using the Quansys Q-View Software™ Version 2.0. The data were compiled and analysed in a custom made macro in Microsoft Excel (Enfer Multiplex Macro, Version 1.0.1.0).

### Anigen lateral flow testing of sera

A selection of serum samples were tested according to the manufacturers instructions using the lateral flow test kit from Anigen (Korea). Following warming to room temperature, 4 drops of the sera were added to the sample deposition area on the lateral flow cassette. The test was read after 20 minutes. Only tests with a positive control line were considered for further evaluation.

### RESULTS

### Development of the multiplex chemiluminescent ELISA

Preliminary studies were conducted to optimize and obtain uniform binding of each antigen using a fluorescent tag. Following optimization of spotting, tests were performed to determine lot to lot variability in pixel intensity at Quansys Biosciences based on the fluorescent tag incorporated into the printing buffer. Plates were tested and evaluated by Enfer for signal variation using a panel of serum samples. Testing of the plates with the detection antibody, with and without sera from a panel of control sera from uninfected cattle, showed that background noise in the system in areas of the surface not coated with antigens was on an average 779.3 relative light units (RLU). Optimisation of the printing concentration for all antigens was carried out on a series of plates printed based on a cohort of control sera. Optimal printing concentrations were determined and used in subsequent testing. Buffers, blocking agents and assay conditions were further optimised on the multiplex assay as well as optimisation of the exposure time for signal capture. The optimal exposure time was determined to be 45 seconds, this giving the best signal to noise ratios with a cohort of samples with varying signal intensities (data not shown).

A total of 1489 TB negative and 522 TB positive animal sera were screened against the antigens listed in Figures 2b (i) and 2b (ii). The individual specificity and sensitivity of all antigens were calculated based on a range of cut offs before selecting individual thresholds for each individual antigen. The individual sensitivities of the antigens ranged from 5.4 - 95.0 % while individual specificities ranged from 69.1 - 99.1%. Results for the overall sensitivity and specificity of the assay are shown in table 3. Calculation of results was based on a combinational approach to the data analysis on results from all antigens for each sample. The individual specificities and sensitivities for ESAT-6 and CFP-10 antigens are shown for these test groups (Table 3).

Table 3 Cohorts of samples tested by Enfer Multiplex assay platform, the Anigen lateral flow kit and figures for individual responses on ESAT-6 and CFP-10 recombinant antigens (data extracted from the multiplex assay for these individual antigens) shown here.

**Table 3**

| Test | n, TB Positive animals | Sensitivity (%) | n, TB negative animals | Specificity (%) |
|---|---|---|---|---|
| Enfer Multiplex assay | **522** | **93.1** | **1489** | **98.4** |
| ESAT-6 | 522 | 40.6 | 1489 | 86.6 |
| CFP-10 | 522 | 82.6 | 1489 | 69.7 |
| Anigen Lateral Flow | 214 | 83.6 | 79 | 83.0* |

| | | | | |
|---|---|---|---|---|
| *reported accuracy of the Anigen lateral flow kit is 85.5% | | | | |

### Screening of infectivity study samples

A total of 5 animals that were challenged with a dose of approximately 5,000 CFU *Mbv* were tested on the Enfer multiplex assay as described in the methods above. Serum samples were tested from the animals at the following time points, pre-challenge time zero and then 2, 5, 10 and 17 weeks post challenge. All animals were determined as having been positively infected by 10 weeks post infection. Three of the 5 animals were detected at 5 weeks.

Table 4 shows the results for this series of samples from the animals over the course of the infectivity study. The time points at which each animal was determined as being positive on the Enfer multiplex ELISA, as well as results for ESAT-6 and CFP-10 antigens are shown.

Table 4. Serum samples from infectivity study (A-E) at times points: pre-challenge, 2, 5, 10 and 17 weeks post challenge. Sample grading is based on the number of antigens used in the Enfer multiplex immunoassay giving a positive response. The individual positive responses for ESAT-6 and CFP-10 antigens (data extracted from the multiplex assay for these individual antigens) are also shown for comparison.

| Sample | week number | Enfer Multiplex immunoassay | ESAT-6 | CFP-10 |
|---|---|---|---|---|
| A | 0 | - | - | - |
| A | 2 | - | - | - |
| A | 5 | ++ | - | - |
| A | 10 | ++ | - | - |
| A | 17 | +++ | - | - |
| B | 0 | - | - | - |
| B | 2 | + | - | + |
| B | 5 | - | - | - |
| B | 10 | + | - | - |
| B | 17 | +++ | - | + |
| C | 0 | - | - | - |
| C | 2 | - | - | - |
| C | 5 | - | - | - |
| C | 10 | + | + | - |
| C | 17 | ++++ | + | + |
| D | 0 | - | - | - |
| D | 2 | - | - | - |
| D | 5 | ++ | - | - |
| D | 10 | +++ | - | - |
| D | 17 | ++ | - | - |
| E | 0 | - | - | - |
| E | 2 | - | - | - |
| E | 5 | ++ | - | - |
| E | 10 | ++ | - | + |
| E | 17 | +++ | - | - |

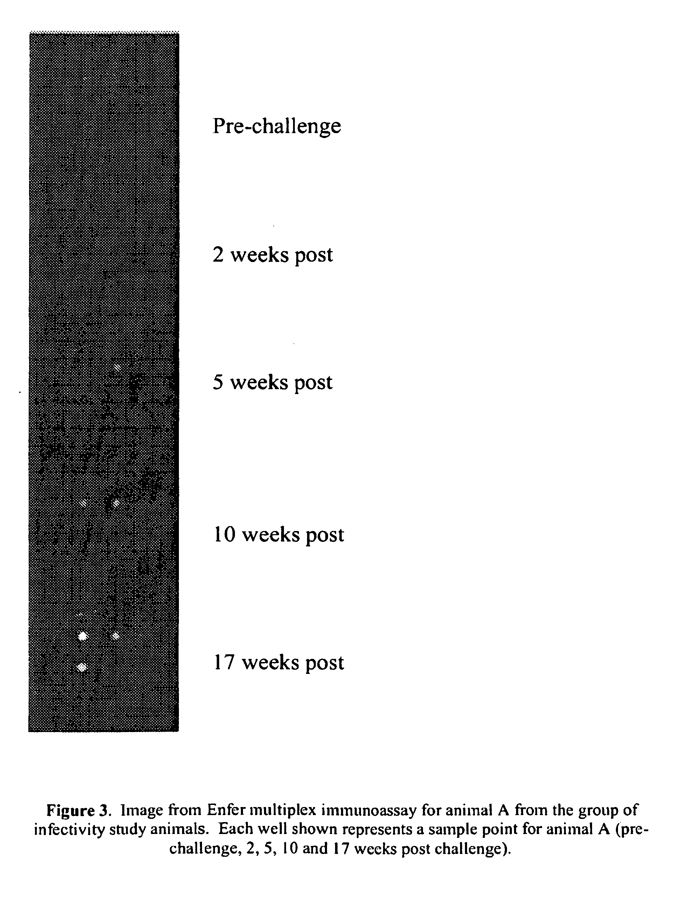

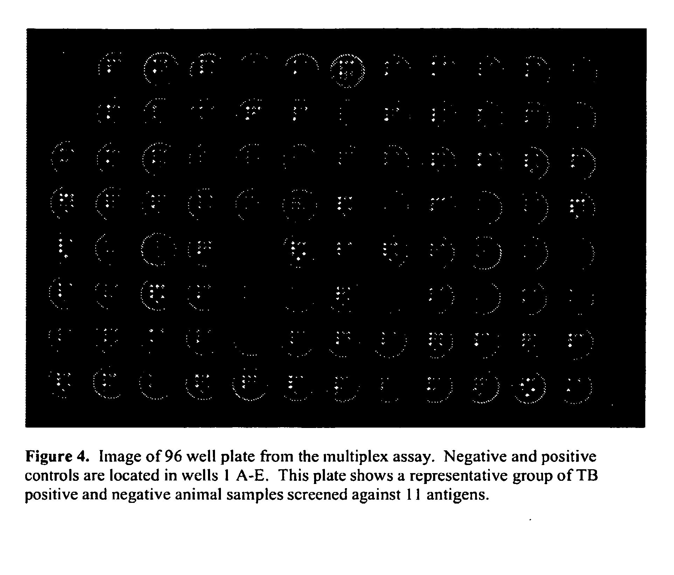

Figure 3 shows an example of the captured image of the test wells from the multiplex ELISA for one of the animals from the infectivity study. Figure 4 illustrates Image of 96 well plate from the multiplex assay. Negative and positive controls are located in wells 1 A-E. This plate shows a representative group of TB positive and negative animal samples screened against 11 antigens.

### Comparison of anti-mortem tests to post mortem tests

The results for the selection of samples tested on the Anigen lateral flow cassette were compiled alongside the data from the Enfer Multiplex Immunoassay and histopathological/bacteriological examination results. To calculate the sensitivity of the tests a maximum of 522 animals were considered that had confirmed histopathological/bacteriological data. Analysis of results from the Enfer Multiplex Immunoassay had a specificity of 98.4% (1489 confirmed negative animals), with a sensitivity of 93.1% for the sera tested. When the same comparison was made for the Anigen lateral flow kit, which has a reported accuracy of greater or equal to 85.5%, a tested specificity of 84.2% (79 negative animal samples tested) and a sensitivity of 84% was observed with the kit (214 positive animals tested). The Anigen lateral flow test kit uses a recombinant MPB70 antigen for detection of antibodies.

### Example 3 Identification of Panel of Antigens for Diagnosis of TB in bovines

The inventors used the multiplex platform described above to analyse antigen expression from 522 cattle, which had been confirmed TB positive by histopathology and culture methods, using 1489 cattle, which had been confirmed TB negative using tuberculin skin tests, as the controls. The inventors identified a panel of five antigens which when assayed together enable diagnosis of tuberculosis infection with very high sensitivity and selectivity. The assaying for the presence of at least two of the five antigens ESAT-6+Rv3616c+pep1(MPB70)+MPB83+MPB70 gave a sensitivity of 93.7%, compared to 71.4% obtained with ESAT-6+Rv3616c+pep1(MPB70)+MPB83, 25.5% obtained using ESAT-6+Rv3616c +pep1(MPB70). The specificity obtained with ESAT-6+Rv3616c+pep1(MPB70)+MPB83+MPB70 was 97.6%.

### Example 4 Identification of Panel of Antigens for Diagnosis of TB in badgers

The inventors used the multiplex platform described above to analyse antigen expression from 67 badgers, which had been confirmed TB positive by histopathology and culture methods, using 133 badgers, which had been confirmed TB negative using tuberculin skin tests, as the controls. The inventors identified a panel of three antigens which when assayed together enable diagnosis of tuberculosis infection with sensitivity and selectivity at levels hitherto not obtained with badgers. The assaying for the presence of at least one of the three antigens Rv3616c+ MPB83+ MPB70 gave a sensitivity of 58.2%, compared to 52.2% obtained with Rv3616c+ MPB70 alone, and 31.3% obtained using MPB70 alone. The specificity obtained with Rv3616c+ MPB83+ MPB70 was 96.2%.

The inventors have developed a robust rapid diagnostic assay for Mbv infection using a multiplex system that can simultaneously detect and analyze antibody activity to multiple antigens. For example, up to 25 antigens can be spotted in a grid format in a single well in a 96 well format. The format can be configured for a larger array of antigens if needed. The assay is rapid and is designed for use with a single plate or use in an automated system with the potential for high throughput. Importantly, software has been developed for image capture and rapid analysis of large numbers of serum samples.

Attempts in the past to develop a robust assay system based on the bovine humoral immune response to bTB have been unsuccessful due to a lack of sensitivity and specificity of the assays. This is in part attributable to variations in the antibody response to Mbv antigens at different stages of infection. The use of a multiplex assay that contains a combination of Mbv specific proteins representative of different stages of disease optimizes the ability to diagnose of this complex disease As reported here the inventors have succeeded in identifying a combination of immune dominant proteins capable of providing a rapid and accurate diagnosis of bTB (bovine TB) status. The inventors have obtained a sensitivity of 93% and specificity of 98% with banks of known negative and positive sera. The sensitivity and specificity was higher than that obtained with a commercial lateral flow assay and an ELISA. They were able to detect antibody activity at dilutions up to 1/250, with the optimized coating concentrations for the antigens and the optimized assay reagents and multiplex platform.

Due to the inclusion of 20 antigens of importance in this multiplex immunoassay the testing of other species is possible on the same platform. Indeed , the inventors have identified particular combinations of antigens which may be used to diagnose the presence of TB in badgers.

Various modifications and variations to the described embodiments of the inventions will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes of carrying out the invention which are obvious to those skilled in the art are intended to be covered by the present invention.

## Claims

1. An assay method for the detection of the presence of Mycobacterium, for example *Mycobacterium bovis,* in a biological sample, said method comprising the steps:
providing a biological sample from an animal; and
determining in vitro the presence or absence of, or the presence or absence of an immune response to, each member of the group of antigens comprising: an Rv3616 antigen, a full length MPB70 protein antigen, an MPB70 linear epitope antigen, an MPB83 antigen, and an ESAT 6 antigen;
wherein the identification of the presence of two or more of said antigens or of an immune response thereto is indicative of the presence of Mycobacterium in the biological sample.

2. The method according to claim 1,
wherein the identification of the presence of two or more of said antigens of said group of antigens, or of an immune response thereto, is indicative of the presence of tuberculosis infection in the animal.

3. The method according to claim 1 or claim 2, further comprising determining the
presence or absence of at least one of the antigens, or an immune response to at least one of the antigens, of the group consisting of an Rv1573 antigen, an Rv1580c antigen and an Rv1585c antigen.

4. A method of diagnosis of tuberculosis in a mammal, for example a reservoir mammal, the method comprising the steps:
providing a biological sample from said animal; and
determining in vitro the presence or absence of, or the presence or absence of an immune response to, each member of the group of antigens comprising an Rv3616 antigen, a full length MPB70 protein antigen, an MPB70 linear epitope antigen, and an MPB83 antigen;
wherein the identification of the presence of one or more of said antigens of said group of antigens, or of an immune response thereto, is indicative of the presence of tuberculosis infection in the mammal.

5. The method according to any one of the preceding claims wherein the presence of said one or more antigens is determined by (i) determining the presence of an antibody response to said antigens, or (ii) determining the presence of a nucleic acid encoding said antigen or by determining the presence of a nucleic acid encoding an antibody to said antigen.

6. A diagnostic kit for the diagnosis of the presence of tuberculosis in a subject, wherein said kit:
(a) comprises an Rv3616 antigen, a full length MPB70 protein antigen, an MPB70 linear epitope antigen, an MPB83 antigen, and an ESAT 6 antigen; or
(b) comprises an antibody molecule with binding specificity for an Rv3616 antigen, an antibody molecule with binding specificity for a conformational epitope of a full length MPB70 protein antigen, an antibody molecule with binding specificity for an MPB70 linear epitope antigen, an antibody molecule with binding specificity for an MPB83 antigen, and an antibody molecule with binding specificity for an ESAT6 antigen; or
(c) is a kit for reservoir animals and comprises an Rv3616 antigen, a full length MPB70 protein antigen, an MPB70 linear epitope antigen, and an MPB83 antigen; or
(d) is a kit for reservoir animals and comprises an antibody molecule with binding specificity for an Rv3616 antigen, an antibody molecule with binding specificity for a conformational epitope of a full length MPB70 protein antigen, an antibody molecule with binding specificity for an MPB70 linear epitope antigen, and an antibody molecule with binding specificity for an MPB83 antigen.

7. The kit according to claim 6, wherein
(i) the kit is kit (a) and said kit further comprises at least one of the antigens selected from the group consisting of an Rv1573 antigen, an Rv1580c antigen and an Rv1585c antigen; or
(ii) the kit is kit (b) and said kit further comprises at least one of the antibody molecules selected from the group consisting of an antibody molecule with binding specificity for an Rv1573 antigen, an antibody molecule with binding specificity for an Rv1580c antigen, and an antibody molecule with binding specificity for an Rv1585c antigen.

8. A vaccine for reservoir animals, said vaccine comprising an Rv3616 antigen, a full length MPB70 protein antigen, an MPB70 linear epitope antigen and an MPB83 antigen.

## Patentansprüche

1. Ein Assay-Verfahren zum Nachweis der Anwesenheit von Mycobacterium, beispielsweise *Mycobacterium bovis,* in einer biologischen Probe, wobei das Verfahren die folgenden Schritte beinhaltet:
Bereitstellen einer biologischen Probe von einem Tier; und
Bestimmen, in vitro, der Anwesenheit oder Abwesenheit von jedem Element der Gruppe von Antigenen, beinhaltend: ein Rv3616-Antigen, ein Volllängen-MPB70-Protein-Antigen, ein lineares MPB70-Epitop-Antigen, ein MPB83-Antigen und ein ESAT-6-Antigen, oder der Anwesenheit oder Abwesenheit einer Immunantwort darauf; wobei die Identifikation der Anwesenheit von zwei oder mehreren der Antigene oder einer Immunantwort darauf auf die Anwesenheit von Mycobacterium in der biologischen Probe hindeutet.

2. Verfahren gemäß Anspruch 1,
wobei die Identifikation der Anwesenheit von zwei oder mehreren der Antigene der Gruppe von Antigenen oder der Immunantwort darauf auf die Anwesenheit einer Tuberkulose-Infektion beim Tier hindeutet.

3. Verfahren gemäß Anspruch 1 oder Anspruch 2,
das ferner das Bestimmen der Anwesenheit oder Abwesenheit von mindestens einem der Antigene oder einer Immunantwort auf mindestens eines der Antigene der Gruppe, bestehend aus einem Rv1573-Antigen, einem Rv1580c-Antigen und einem Rv1585c-Antigen, beinhaltet.

4. Ein Verfahren zur Diagnose von Tuberkulose bei einem Säugetier, zum Beispiel einem Reservoir-Säugetier, wobei das Verfahren die folgenden Schritte beinhaltet:
Bereitstellen einer biologischen Probe von dem Tier; und
Bestimmen, in vitro, der Anwesenheit oder Abwesenheit von jedem Element der Gruppe von Antigenen, beinhaltend ein Rv3616-Antigen, ein Volllängen-MPB70-Protein-Antigen, ein lineares MPB70-Epitop-Antigen und ein MPB83-Antigen, oder der Anwesenheit oder Abwesenheit einer Immunantwort darauf;
wobei die Identifikation der Anwesenheit von einem oder mehreren der Antigene der Gruppe von Antigenen oder einer Immunantwort darauf auf die Anwesenheit einer Tuberkulose-Infektion beim Säugetier hindeutet.

5. Verfahren gemäß einem der vorhergehenden Ansprüche, wobei die Anwesenheit des einen oder der mehreren Antigene bestimmt wird durch (i) das Bestimmen der Anwesenheit einer Antikörper-Antwort auf die Antigene oder (ii) das Bestimmen der Anwesenheit einer Nukleinsäure, die das Antigen codiert, oder durch das Bestimmen der Anwesenheit einer Nukleinsäure, die einen Antikörper gegen das Antigen codiert.

6. Ein Diagnosekit zur Diagnose der Anwesenheit von Tuberkulose bei einem Individuum, wobei das Kit:
(a) ein Rv3616-Antigen, ein Volllängen-MPB70-Protein-Antigen, ein lineares MPB70-Epitop-Antigen, ein MPB83-Antigen und ein ESAT-6-Antigen beinhaltet; oder
(b) ein Antikörper-Molekül mit Bindungsspezifität für ein Rv3616-Antigen, ein Antikörper-Molekül mit Bindungsspezifität für ein Konformationsepitop eines Volllängen-MPB70-Protein-Antigens, ein Antikörper-Molekül mit Bindungsspezifität für ein lineares MPB70-Epitop-Antigen, ein Antikörper-Molekül mit Bindungsspezifität für ein MPB83-Antigen und ein Antikörper-Molekül mit Bindungsspezifität für ein ESAT6-Antigen beinhaltet; oder
(c) ein Kit für Reservoir-Tiere ist und ein Rv3616-Antigen, ein Volllängen-MPB70-Protein-Antigen, ein lineares MPB70-Epitop-Antigen und ein MPB83-Antigen beinhaltet; oder
(d) ein Kit für Reservoir-Tiere ist und ein Antikörper-Molekül mit Bindungsspezifität für ein Rv3616-Antigen, ein Antikörper-Molekül mit Bindungsspezifität für ein Konformationsepitop eines Volllängen-MPB70-Protein-Antigens, ein Antikörper-Molekül mit Bindungsspezifität für ein lineares MPB70-Epitop-Antigen und ein Antikörper-Molekül mit Bindungsspezifität für ein MPB83-Antigen beinhaltet.

7. Kit gemäß Anspruch 6, wobei
(i) das Kit Kit (a) ist und das Kit ferner mindestens eines der Antigene beinhaltet, die ausgewählt sind aus der Gruppe, bestehend aus einem Rv1573-Antigen, einem Rv1580c-Antigen und einem Rv1585c-Antigen; oder
(ii) das Kit Kit (b) ist und das Kit ferner mindestens eines der Antikörper-Moleküle beinhaltet, die ausgewählt sind aus der Gruppe, bestehend aus einem Antikörper-Molekül mit Bindungsspezifität für ein Rv1573-Antigen, einem Antikörper-Molekül mit Bindungsspezifität für ein Rv1580c-Antigen und ein Antikörper-Molekül mit Bindungsspezifität für ein Rv1585c-Antigen.

8. Ein Impfstoff für Reservoir-Tiere, wobei der Impfstoff ein Rv3616-Antigen, ein Volllängen-MPB70-Protein-Antigen, ein lineares MPB70-Epitop-Antigen und ein MPB83-Antigen beinhaltet.

## Revendications

1. Une méthode d'essai pour la détection de la présence de Mycobacterium, par exemple *Mycobacterium bovis,* dans un échantillon biologique, ladite méthode comprenant les étapes suivantes :
fourniture d'un échantillon biologique prélevé sur un animal ; et
détermination in vitro de la présence ou l'absence de, ou la présence ou l'absence d'une réponse immunitaire à, chaque membre du groupe d'antigènes comprenant :
un antigène Rv3616, un antigène protéique MPB70 de longueur totale, un antigène dont l'épitope est un épitope linéaire MPB70, un antigène MPB83 et un antigène ESAT 6 ;
dans lequel l'identification de la présence de deux, ou plus, desdits antigènes ou d'une réponse immunitaire à ceux-ci est indicatrice de la présence de Mycobacterium dans l'échantillon biologique.

2. La méthode selon la revendication 1, dans laquelle l'identification de la présence de deux, ou plus, desdits antigènes dudit groupe d'antigènes, ou d'une réponse immunitaire à ceux-ci, est indicatrice de la présence de l'infection à tuberculose chez l'animal.

3. La méthode selon la revendication 1 ou la revendication 2, comprenant en sus la détermination de la présence ou l'absence d'au moins l'un des antigènes, ou d'une réponse immunitaire à au moins l'un des antigènes, du groupe consistant en un antigène Rv1573, un antigène Rv1580c et un antigène Rv1585c.

4. Une méthode de diagnostic de la tuberculose chez un mammifère, par exemple un mammifère réservoir, la méthode comprenant les étapes suivantes :
fourniture d'un échantillon biologique prélevé sur ledit animal ; et
détermination in vitro de la présence ou l'absence de, ou la présence ou l'absence d'une réponse immunitaire à, chaque membre du groupe d'antigènes comprenant un antigène Rv3616, un antigène protéique MPB70 de longueur totale, un antigène dont l'épitope est un épitope linéaire MPB70 et un antigène MPB83 ;
dans laquelle l'identification de la présence d'un ou plusieurs desdits antigènes dudit groupe d'antigènes, ou d'une réponse immunitaire à ceux-ci, est indicatrice de la présence de l'infection à tuberculose chez le mammifère.

5. La méthode selon une quelconque des revendications précédentes dans laquelle la présence d'un ou plusieurs desdits antigènes est déterminée par (i) la détermination de la présence d'une réponse en anticorps audits antigènes, ou (ii) la détermination de la présence d'un acide nucléique codant pour ledit antigène ou par la détermination de la présence d'un acide nucléique codant pour un anticorps audit antigène.

6. Une trousse de diagnostic pour le diagnostic de la présence de tuberculose chez un sujet, dans laquelle ladite trousse :
(a) comprend un antigène Rv3616, un antigène protéique MPB70 de longueur totale, un antigène dont l'épitope est un épitope linéaire MPB70, un antigène MPB83 et un antigène ESAT 6 ; ou
(b) comprend une molécule d'anticorps présentant une spécificité de liaison pour un antigène Rv3616, une molécule d'anticorps présentant une spécificité de liaison pour un épitope conformationnel d'un antigène protéique MPB70 de longueur totale, une molécule d'anticorps présentant une spécificité de liaison pour un antigène dont l'épitope est un épitope linéaire MPB70, une molécule d'anticorps présentant une spécificité de liaison pour un antigène MPB83, et une molécule d'anticorps présentant une spécificité de liaison pour un antigène ESAT 6 ; ou
(c) est une trousse pour animaux réservoirs et comprend un antigène Rv3616, un antigène protéique MPB70 de longueur totale, un antigène dont l'épitope est un épitope linéaire MPB70, et un antigène MPB83 ; ou
(d) est une trousse pour animaux réservoirs et comprend une molécule d'anticorps présentant une spécificité de liaison pour un antigène Rv3616, une molécule d'anticorps présentant une spécificité de liaison pour un épitope conformationnel d'un antigène protéique MPB70 de longueur totale, une molécule d'anticorps présentant une spécificité de liaison pour un antigène dont l'épitope est un épitope linéaire MPB70, et une molécule d'anticorps présentant une spécificité de liaison pour un antigène MPB83.

7. La trousse selon la revendication 6, dans laquelle
(i) la trousse est la trousse (a) et ladite trousse comprend en sus au moins l'un des antigènes choisis dans le groupe consistant en un antigène Rv1573, un antigène Rv1580c et un antigène Rv1585c ; ou
(ii) la trousse est la trousse (b) et ladite trousse comprend en sus au moins l'une des molécules d'anticorps choisies dans le groupe consistant en une molécule d'anticorps présentant une spécificité de liaison pour un antigène Rv1573, une molécule d'anticorps présentant une spécificité de liaison pour un antigène Rv1580c, et une molécule d'anticorps présentant une spécificité de liaison pour un antigène Rv1585c.

8. Un vaccin pour animaux réservoirs, ledit vaccin comprenant un antigène Rv3616, un antigène protéique MPB70 de longueur totale, un antigène dont l'épitope est un épitope linéaire MPB70 et un antigène MPB83.


### SEQUENCE LISTING

<110> Fusion Antibodies Limited
   Enfer Technology Limited
   Olwill, Shane A
   Johnston, James A
   Whelan, Clare
   Clarke, John
   Kwok, Hang Fai
<120> Diagnostic Method and Kit
<130> P102121.WO.01
<150> GB0716070.8
   <151> 2007-08-17
<160> 26
<170> Patent In version 3.3
<210> 1
   <211> 392
   <212> PRT
   <213> Mycobacterium bovis
<400> 1
   Met Ser Arg Ala Phe Ile Ile Asp Pro Thr Ile Ser Ala Ile Asp Gly
   1               5                   10                  15
   Leu Tyr Asp Leu Leu Gly Ile Gly Ile Pro Asn Gln Gly Gly Ile Leu
               20                  25                  30
   Tyr Ser Ser Leu Glu Tyr Phe Glu Lys Ala Leu Glu Glu Leu Ala Ala
           35                  40                  45
   Ala Phe Pro Gly Asp Gly Trp Leu Gly Ser Ala Ala Asp Lys Tyr Ala
       50                  55                  60
   Gly Lys Asn Arg Asn His Val Asn Phe Phe Gln Glu Leu Ala Asp Leu
   65                  70                  75                  80
   Asp Arg Gln Leu Ile Ser Leu Ile His Asp Gln Ala Asn Ala Val Gln
                   85                  90                  95
   Thr Thr Arg Asp Ile Leu Glu Gly Ala Lys Lys Gly Leu Glu Phe Val
               100                 105                 110
   Arg Pro Val Ala Val Asp Leu Thr Tyr Ile Pro Val Val Gly His Ala
           115                 120                 125
   Leu Ser Ala Ala Phe Gln Ala Pro Phe Cys Ala Gly Ala Met Ala Val
   130 135 140
   Val Gly Gly Ala Leu Ala Tyr Leu Val Val Lys Thr Leu Ile Asn Ala
   145 150 155 160
   Thr Gln Leu Leu Lys Leu Leu Ala Lys Leu Ala Glu Leu Val Ala Ala
   165 170 175
   Ala Ile Ala Asp Ile Ile Ser Asp Val Ala Asp Ile Ile Lys Gly Thr
   180 185 190
   Leu Gly Glu Val Trp Glu Phe Ile Thr Asn Ala Leu Asn Gly Leu Lys
   195 200 205
   Glu Leu Trp Asp Lys Leu Thr Gly Trp Val Thr Gly Leu Phe Ser Arg
   210 215 220
   Gly Trp Ser Asn Leu Glu Ser Phe Phe Ala Gly Val Pro Gly Leu Thr
   225 230 235 240
   Gly Ala Thr Ser Gly Leu Ser Gln Val Thr Gly Leu Phe Gly Ala Ala
   245 250 255
   Gly Leu Ser Ala Ser Ser Gly Leu Ala His Ala Asp Ser Leu Ala Ser
   260 265 270
   Ser Ala Ser Leu Pro Ala Leu Ala Gly Ile Gly Gly Gly Ser Gly Phe
   275 280 285
   Gly Gly Leu Pro Ser Leu Ala Gln Val His Ala Ala Ser Thr Arg Gln
   290 295 300
   Ala Leu Arg Pro Arg Ala Asp Gly Pro Val Gly Ala Ala Ala Glu Gln
   305 310 315 320
   Val Gly Gly Gln Ser Gln Leu Val Ser Ala Gln Gly Ser Gln Gly Met
   325 330 335
   Gly Gly Pro Val Gly Met Gly Gly Met His Pro Ser Ser Gly Ala Ser
   340 345 350
   Lys Gly Thr Thr Thr Lys Lys Tyr Ser Glu Gly Ala Ala Ala Gly Thr
   355 360 365
   Glu Asp Ala Glu Arg Ala Pro Val Glu Ala Asp Ala Gly Gly Gly Gln
   370 375 380
   Lys Val Leu Val Arg Asn Val Val
   385 390
<210> 2
   <211> 71
   <212> PRT
   <213> Mycobacterium bovis
<400> 2
   Ala Ser Thr Arg Gln Ala Leu Arg Pro Arg Ala Asp Gly Pro Val Gly
   1 5 10 15
   Ala Ala Ala Glu Gln Val Gly Gly Gln Ser Gln Leu Val Ser Ala Gln
   20 25 30
   Gly Ser Gln Gly Met Gly Gly Pro Val Gly Met Gly Gly Met His Pro
   35 40 45
   Ser Ser Gly Ala Ser Lys Gly Thr Thr Thr Lys Lys Tyr Ser Glu Gly
   50 55 60
   Ala Ala Ala Gly Thr Glu Asp
   65 70
<210> 3
   <211> 60
   <212> PRT
   <213> Mycobacterium bovis
<400> 3
   Ser Leu Glu Tyr Phe Glu Lys Ala Leu Glu Glu Leu Ala Ala Ala Phe
   1 5 10 15
   Pro Gly Asp Gly Trp Leu Gly Ser Ala Ala Asp Lys Tyr Ala Gly Lys
   20 25 30
   Asn Arg Asn His Val Asn Phe Phe Gln Glu Leu Ala Asp Leu Asp Arg
   35 40 45
   Gln Leu Ile Ser Leu Ile His Asp Gln Ala Asn Ala
   50 55 60
<210> 4
   <211> 34
   <212> PRT
   <213> Mycobacterium bovis
<400> 4
   Met Ser Arg Ala Phe Ile Ile Asp Pro Thr Ile Ser Ala Ile Asp Gly
   1 5 10 15
   Leu Tyr Asp Leu Leu Gly Ile Gly Ile Pro Asn Gln Gly Gly Ile Leu
   20 25 30
   Tyr Ser
<210> 5
   <211> 298
   <212> PRT
   <213> Mycobacterium bovis
<400> 5
   Val Gln Thr Thr Arg Asp Ile Leu Glu Gly Ala Lys Lys Gly Leu Glu
   1 5 10 15
   Phe Val Arg Pro Val Ala Val Asp Leu Thr Tyr Ile Pro Val Val Gly
   20 25 30
   His Ala Leu Ser Ala Ala Phe Gln Ala Pro Phe Cys Ala Gly Ala Met
   35 40 45
   Ala Val Val Gly Gly Ala Leu Ala Tyr Leu Val Val Lys Thr Leu Ile
   50 55 60
   Asn Ala Thr Gln Leu Leu Lys Leu Leu Ala Lys Leu Ala Glu Leu Val
   65 70 75 80
   Ala Ala Ala Ile Ala Asp Ile Ile Ser Asp Val Ala Asp Ile Ile Lys
   85 90 95
   Gly Thr Leu Gly Glu Val Trp Glu Phe Ile Thr Asn Ala Leu Asn Gly
   100 105 110
   Leu Lys Glu Leu Trp Asp Lys Leu Thr Gly Trp Val Thr Gly Leu Phe
   115 120 125
   Ser Arg Gly Trp Ser Asn Leu Glu Ser Phe Phe Ala Gly Val Pro Gly
   130 135 140
   Leu Thr Gly Ala Thr Ser Gly Leu Ser Gln Val Thr Gly Leu Phe Gly
   145 150 155 160
   Ala Ala Gly Leu Ser Ala Ser Ser Gly Leu Ala His Ala Asp Ser Leu
   165 170 175
   Ala Ser Ser Ala Ser Leu Pro Ala Leu Ala Gly Ile Gly Gly Gly Ser
   180 185 190
   Gly Phe Gly Gly Leu Pro Ser Leu Ala Gln Val His Ala Ala Ser Thr
   195 200 205
   Arg Gln Ala Leu Arg Pro Arg Ala Asp Gly Pro Val Gly Ala Ala Ala
   210 215 220
   Glu Gln Val Gly Gly Gln Ser Gln Leu Val Ser Ala Gln Gly Ser Gln
   225 230 235 240
   Gly Met Gly Gly Pro Val Gly Met Gly Gly Met His Pro Ser Ser Gly
   245 250 255
   Ala Ser Lys Gly Thr Thr Thr Lys Lys Tyr Ser Glu Gly Ala Ala Ala
   260 265 270
   Gly Thr Glu Asp Ala Glu Arg Ala Pro Val Glu Ala Asp Ala Gly Gly
   275 280 285
   Gly Gln Lys Val Leu Val Arg Asn Val Val
   290 295
<210> 6
   <211> 193
   <212> PRT
   <213> Mycobacterium bovis
<400> 6
   Met Lys Val Lys Asn Thr Ile Ala Ala Thr Ser Phe Ala Ala Ala Gly
   1 5 10 15
   Leu Ala Ala Leu Ala Val Ala Val Ser Pro Pro Ala Ala Ala Gly Asp
   20 25 30
   Leu Val Gly Pro Gly Cys Ala Glu Tyr Ala Ala Ala Asn Pro Thr Gly
   35 40 45
   Pro Ala Ser Val Gln Gly Met Ser Gln Asp Pro Val Ala Val Ala Ala
   50 55 60
   Ser Asn Asn Pro Glu Leu Thr Thr Leu Thr Ala Ala Leu Ser Gly Gln
   65 70 75 80
   Leu Asn Pro Gln Val Asn Leu Val Asp Thr Leu Asn Ser Gly Gln Tyr
                   85                  90                  95
   Thr Val Phe Ala Pro Thr Asn Ala Ala Phe Ser Lys Leu Pro Ala Ser
               100                 105                 110
   Thr Ile Asp Glu Leu Lys Thr Asn Ser Ser Leu Leu Thr Ser Ile Leu
           115                 120                 125
   Thr Tyr His Val Val Ala Gly Gln Thr Ser Pro Ala Asn Val Val Gly
       130                 135                 140
   Thr Arg Gln Thr Leu Gln Gly Ala Ser Val Thr Val Thr Gly Gln Gly
   145                 150                 155                 160
   Asn Ser Leu Lys Val Gly Asn Ala Asp Val Val Cys Gly Gly Val Ser
                   165                 170                 175
   Thr Ala Asn Ala Thr Val Tyr Met Ile Asp Ser Val Leu Met Pro Pro
               180                 185                 190
   Ala
<210> 7
   <211> 220
   <212> PRT
   <213> Mycobacterium bovis
<400> 7
   Met Ile Asn Val Gln Ala Lys Pro Ala Ala Ala Ala Ser Leu Ala Ala
   1               5                   10                  15
   Ile Ala Ile Ala Phe Leu Ala Gly Cys Ser Ser Thr Lys Pro Val Ser
               20                  25                  30
   Gln Asp Thr Ser Pro Lys Pro Ala Thr Ser Pro Ala Ala Pro Val Thr
           35                  40                  45
   Thr Ala Ala Met Ala Asp Pro Ala Ala Asp Leu Ile Gly Arg Gly Cys
       50                  55                  60
   Ala Gln Tyr Ala Ala Gln Asn Pro Thr Gly Pro Gly Ser Val Ala Gly
   65                  70                  75                  80
   Met Ala Gln Asp Pro Val Ala Thr Ala Ala Ser Asn Asn Pro Met Leu
                   85                  90                  95
   Ser Thr Leu Thr Ser Ala Leu Ser Gly Lys Leu Asn Pro Asp Val Asn
               100                 105                 110
   Leu Val Asp Thr Leu Asn Gly Gly Glu Tyr Thr Val Phe Ala Pro Thr
           115                 120                 125
   Asn Ala Ala Phe Asp Lys Leu Pro Ala Ala Thr Ile Asp Gln Leu Lys
       130                 135                 140
   Thr Asp Ala Lys Leu Leu Ser Ser Ile Leu Thr Tyr His Val Ile Ala
   145                 150                 155                 160
   Gly Gln Ala Ser Pro Ser Arg Ile Asp Gly Thr His Gln Thr Leu Gln
                   165                 170                 175
   Gly Ala Asp Leu Thr Val Ile Gly Ala Arg Asp Asp Leu Met Val Asn
               180                 185                 190
   Asn Ala Gly Leu Val Cys Gly Gly Val His Thr Ala Asn Ala Thr Val
           195                 200                 205
   Tyr Met Ile Asp Thr Val Leu Met Pro Pro Ala Gln
       210                 215                 220
<210> 8
   <211> 94
   <212> PRT
   <213> Mycobacterium bovis
<400> 8
   Thr Glu Gln Gln Trp Asn Phe Ala Gly Ile Glu Ala Ala Ala Ser Ala
   1               5                   10                  15
   Ile Gln Gly Asn Val Thr Ser Ile His Ser Leu Leu Asp Glu Gly Lys
               20                  25                  30
   Gln Ser Leu Thr Lys Leu Ala Ala Ala Trp Gly Gly Ser Gly Ser Glu
           35                  40                  45
   Ala Tyr Gln Gly Val Gln Gln Lys Trp Asp Ala Thr Ala Thr Glu Leu
       50                  55                  60
   Asn Asn Ala Leu Gln Asn Leu Ala Arg Thr Ile Ser Glu Ala Gly Gln
   65                  70                  75                  80
   Ala Met Ala Ser Thr Glu Gly Asn Val Thr Gly Met Phe Ala
   85 90
<210> 9
   <211> 99
   <212> PRT
   <213> Mycobacterium bovis
<400> 9
   Ala Glu Met Lys Thr Asp Ala Ala Thr Leu Ala Gln Glu Ala Gly Asn
   1 5 10 15
   Phe Glu Arg Ile Ser Gly Asp Leu Lys Thr Gln Ile Asp Gln Val Glu
   20 25 30
   Ser Thr Ala Gly Ser Leu Gln Gly Gln Trp Arg Gly Ala Ala Gly Thr
   35 40 45
   Ala Ala Gln Ala Ala Val Val Arg Phe Gln Glu Ala Ala Asn Lys Gln
   50 55 60
   Lys Gln Glu Leu Asp Glu Ile Ser Thr Asn Ile Arg Gln Ala Gly Val
   65 70 75 80
   Gln Tyr Ser Arg Ala Asp Glu Glu Gln Gln Gln Ala Leu Ser Ser Gln
   85 90 95
   Met Gly Phe
<210> 10
   <211> 157
   <212> PRT
   <213> Mycobacterium bovis
<400> 10
   Met Asn Asn Leu Ala Leu Trp Ser Arg Pro Val Trp Asp Val Glu Pro
   1 5 10 15
   Trp Asp Arg Trp Leu Arg Asp Phe Phe Gly Pro Ala Ala Thr Thr Asp
   20 25 30
   Trp Tyr Arg Pro Val Ala Gly Asp Phe Thr Pro Ala Ala Glu Ile Val
   35 40 45
   Lys Asp Gly Asp Asp Ala Val Val Arg Leu Glu Leu Pro Gly Ile Asp
   50 55 60
   Val Asp Lys Asn Val Glu Leu Asp Pro Gly Gln Pro Val Ser Arg Leu
   65 70 75 80
   Val Ile Arg Gly Glu His Arg Asp Glu His Thr Gln Asp Ala Gly Asp
   85 90 95
   Lys Asp Gly Arg Thr Leu Arg Glu Ile Arg Tyr Gly Ser Phe Arg Arg
   100 105 110
   Ser Phe Arg Leu Pro Ala His Val Thr Ser Glu Ala Ile Ala Ala Ser
   115 120 125
   Tyr Asp Ala Gly Val Leu Thr Val Arg Val Ala Gly Ala Tyr Lys Ala
   130 135 140
   Pro Ala Glu Thr Gln Ala Gln Arg Ile Ala Ile Thr Lys
   145 150 155
<210> 11
   <211> 99
   <212> PRT
   <213> Mycobacterium bovis
<400> 11
   Met Glu Lys Met Ser His Asp Pro Ile Ala Ala Asp Ile Gly Thr Gln
   1 5 10 15
   Val Ser Asp Asn Ala Leu His Gly Val Thr Ala Gly Ser Thr Ala Leu
   20 25 30
   Thr Ser Val Thr Gly Leu Val Pro Ala Gly Ala Asp Glu Val Ser Ala
   35 40 45
   Gln Ala Ala Thr Ala Phe Thr Ser Glu Gly Ile Gln Leu Leu Ala Ser
   50 55 60
   Asn Ala Ser Ala Gln Asp Gln Leu His Arg Ala Gly Glu Ala Val Gln
   65 70 75 80
   Asp Val Ala Arg Thr Tyr Ser Gln Ile Asp Asp Gly Ala Ala Gly Val
   85 90 95
   Phe Ala Glu
<210> 12
   <211> 368
   <212> PRT
   <213> Mycobacterium bovis
<400> 12
   Met Leu Trp His Ala Met Pro Pro Glu Leu Asn Thr Ala Arg Leu Met
   1               5                   10                  15
   Ala Gly Ala Gly Pro Ala Pro Met Leu Ala Ala Ala Ala Gly Trp Gln
               20                  25                  30
   Thr Leu Ser Ala Ala Leu Asp Ala Gln Ala Val Glu Leu Thr Ala Arg
           35                  40                  45
   Leu Asn Ser Leu Gly Glu Ala Trp Thr Gly Gly Gly Ser Asp Lys Ala
       50                  55                  60
   Leu Ala Ala Ala Thr Pro Met Val Val Trp Leu Gln Thr Ala Ser Thr
   65                  70                  75                  80
   Gln Ala Lys Thr Arg Ala Met Gln Ala Thr Ala Gln Ala Ala Ala Tyr
                   85                  90                  95
   Thr Gln Ala Met Ala Thr Thr Pro Ser Leu Pro Glu Ile Ala Ala Asn
               100                 105                 110
   His Ile Thr Gln Ala Val Leu Thr Ala Thr Asn Phe Phe Gly Ile Asn
           115                 120                 125
   Thr Ile Pro Ile Ala Leu Thr Glu Met Asp Tyr Phe Ile Arg Met Trp
       130                 135                 140
   Asn Gln Ala Ala Leu Ala Met Glu Val Tyr Gln Ala Glu Thr Ala Val
   145                 150                 155                 160
   Asn Thr Leu Phe Glu Lys Leu Glu Pro Met Ala Ser Ile Leu Asp Pro
                   165                 170                 175
   Gly Ala Ser Gln Ser Thr Thr Asn Pro Ile Phe Gly Met Pro Ser Pro
               180                 185                 190
   Gly Ser Ser Thr Pro Val Gly Gln Leu Pro Pro Ala Ala Thr Gln Thr
           195                 200                 205
   Leu Gly Gln Leu Gly Glu Met Ser Gly Pro Met Gln Gln Leu Thr Gln
   210 215 220
   Pro Leu Gln Gln Val Thr Ser Leu Phe Ser Gln Val Gly Gly Thr Gly
   225 230 235 240
   Gly Gly Asn Pro Ala Asp Glu Glu Ala Ala Gln Met Gly Leu Leu Gly
   245 250 255
   Thr Ser Pro Leu Ser Asn His Pro Leu Ala Gly Gly Ser Gly Pro Ser
   260 265 270
   Ala Gly Ala Gly Leu Leu Arg Ala Glu Ser Leu Pro Gly Ala Gly Gly
   275 280 285
   Ser Leu Thr Arg Thr Pro Leu Met Ser Gln Leu Ile Glu Lys Pro Val
   290 295 300
   Ala Pro Ser Val Met Pro Ala Ala Ala Ala Gly Ser Ser Ala Thr Gly
   305 310 315 320
   Gly Ala Ala Pro Val Gly Ala Gly Ala Met Gly Gln Gly Ala Gln Ser
   325 330 335
   Gly Gly Ser Thr Arg Pro Gly Leu Val Ala Pro Ala Pro Leu Ala Gln
   340 345 350
   Glu Arg Glu Glu Asp Asp Glu Asp Asp Trp Asp Glu Glu Asp Asp Trp
   355 360 365
<210> 13
   <211> 136
   <212> PRT
   <213> Mycobacterium bovis
<400> 13
   Met Thr Thr Thr Pro Ala Arg Phe Asn His Leu Val Thr Val Thr Asp
   1 5 10 15
   Leu Glu Thr Gly Asp Arg Ala Val Cys Asp Arg Asp Gln Val Ala Glu
   20 25 30
   Thr Ile Arg Ala Trp Phe Pro Asp Ala Pro Leu Glu Val Arg Glu Ala
   35 40 45
   Leu Val Arg Leu Gln Ala Ala Leu Asn Arg His Glu His Thr Gly Glu
   50 55 60
   Leu Glu Ala Phe Leu Arg Ile Ser Val Glu His Ala Asp Ala Ala Gly
   65                  70                  75                  80
   Gly Asp Glu Cys Gly Pro Ala Ile Leu Ala Gly Arg Ser Gly Pro Glu
                   85                  90                  95
   Gln Ala Ala Ile Asn Arg Gln Leu Gly Leu Ala Gly Asp Asp Glu Pro
               100                 105                 110
   Asp Gly Asp Asp Thr Pro Pro Trp Ser Arg Met Ile Gly Leu Gly Gly
           115                 120                 125
   Gly Ser Pro Ala Glu Asp Glu Arg
       130                 135
<210> 14
   <211> 90
   <212> PRT
   <213> Mycobacterium bovis
<400> 14
   Met Ala Glu Thr Pro Asp His Ala Glu Leu Arg Arg Arg Ile Ala Asp
   1               5                   10                  15
   Met Ala Phe Asn Ala Asp Val Gly Met Ala Thr Cys Lys Arg Cys Gly
               20                  25                  30
   Asp Ala Val Pro Tyr Ile Ile Leu Pro Asn Leu Gln Thr Gly Glu Pro
           35                  40                  45
   Val Met Gly Val Ala Asp Asn Lys Trp Lys Arg Ala Asn Cys Pro Val
       50                  55                  60
   Asp Val Gly Lys Pro Cys Pro Phe Leu Ile Ala Glu Gly Val Ala Asp
   65                  70                  75                  80
   Ser Thr Asp Asp Thr Ile Glu Val Asp Gln
                   85                  90
<210> 15
   <211> 171
   <212> PRT
   <213> Mycobacterium bovis
<400> 15
   Met Ser Arg His His Asn Ile Val Ile Val Cys Asp His Gly Arg Lys
   1               5                   10                  15
   Gly Asp Gly Arg Ile Glu His Glu Arg Cys Asp Leu Val Ala Pro Ile
               20                  25                  30
   Ile Trp Val Asp Glu Thr Gln Gly Trp Leu Pro Gln Ala Pro Ala Val
           35                  40                  45
   Ala Thr Leu Leu Asp Asp Asp Asn Gln Pro Arg Ala Val Ile Gly Leu
       50                  55                  60
   Pro Pro Asn Glu Ser Arg Leu Arg Pro Glu Met Arg Arg Asp Gly Trp
   65                  70                  75                  80
   Val Arg Leu His Trp Glu Phe Ala Cys Leu Arg Tyr Gly Ala Ala Gly
                   85                  90                  95
   Val Arg Thr Cys Glu Gln Arg Pro Val Arg Val Arg Asn Gly Asp Leu
               100                 105                 110
   Gln Thr Leu Cys Glu Asn Val Pro Arg Leu Leu Thr Gly Leu Ala Gly
           115                 120                 125
   Asn Pro Asp Tyr Ala Pro Gly Phe Ala Val Gln Ser Asp Ala Val Val
       130                 135                 140
   Val Ala Met Trp Leu Trp Arg Thr Leu Cys Glu Ser Asp Thr Pro Asn
   145                 150                 155                 160
   Lys Leu Arg Ala Thr Pro Thr Arg Gly Ser Cys
                   165                 170
<210> 16
   <211> 16
   <212> PRT
   <213> Mycobacterium bovis
<400> 16
   Lys Trp Asp Ala Thr Ala Thr Glu Leu Asn Asn Ala Leu Gln Asn Leu
   1               5                   10                  15
<210> 17
   <211> 23
   <212> PRT
   <213> Artificial
<400> 17
   Lys Gly Ser Gly Ser Asn Ile Arg Gln Ala Gly Val Gln Tyr Ser Arg
   1 5 10 15
   Ala Asp Glu Glu Gln Gln Gln
   20
<210> 18
   <211> 22
   <212> PRT
   <213> Artificial
<400> 18
   Lys Gly Ser Gly Ser Met Ala Glu Met Lys Thr Asp Ala Ala Thr Leu
   1 5 10 15
   Ala Gln Glu Ala Gly Asn
   20
<210> 19
   <211> 25
   <212> PRT
   <213> Artificial
<400> 19
   Lys Gly Ser Gly Ser Ser Val Gln Gly Met Ser Gln Asp Pro Val Ala
   1 5 10 15
   Val Ala Ala Ser Asn Asn Pro Glu Leu
   20 25
<210> 20
   <211> 28
   <212> PRT
   <213> Artificial
<400> 20
   Lys Glu Lys Asp Lys Asp Gly Asn Gly Leu Val Cys Gly Gly Val His
   1 5 10 15
   Thr Ala Asn Ala Thr Val Tyr Met Ile Asp Thr Val
   20 25
<210> 21
   <211> 18
   <212> PRT
   <213> Artificial
<400> 21
   Asp Lys Gly Asp Gly Ser Ala Pro Lys Thr Tyr Cys Glu Glu Leu Lys
   1 5 10 15
   Gly Thr
<210> 22
   <211> 17
   <212> PRT
   <213> Mycobacterium bovis
<400> 22
   Gly Val Ala Asp Asn Lys Trp Lys Arg Ala Asn Cys Pro Val Asp Val
   1               5                   10                  15
   Gly
<210> 23
   <211> 25
   <212> PRT
   <213> Artificial
<400> 23
   Lys Gly Ser Gly Ser Ser Val Gln Gly Met Ser Gln Asp Pro Val Ala
   1               5                   10                  15
   Val Ala Ala Ser Asn Asn Pro Glu Leu
               20                  25
<210> 24
   <211> 22
   <212> PRT
   <213> Artificial
<400> 24
   Lys Gly Ser Gly Ser Met Ala Glu Met Lys Thr Asp Ala Ala Thr Leu
   1               5                   10                  15
   Ala Gln Glu Ala Gly Asn
               20
<210> 25
   <211> 23
   <212> PRT
   <213> Artificial
<400> 25
   Lys Gly Ser Gly Ser Asn Ile Arg Gln Ala Gly Val Gln Tyr Ser Arg
   1               5                   10                  15
   Ala Asp Glu Glu Gln Gln Gln
               20
<210> 26
   <211> 18
   <212> PRT
   <213> Mycobacterium bovis
<400> 26
   Val Val Arg Phe Gln Glu Ala Ala Asn Lys Gln Lys Gln Glu Leu Asp
   1               5                   10                  15
   Glu Ile